# EUROPEAN PATENT APPLICATION

(11) **EP 4 628 594 A1**
(43) Date of publication of application: **08.10.2025**
(21) Application number: 24168844.9
(22) Date of filing: 05.04.2024
(51) Int. Cl.: C12Q 1/6848

(54) **METHODS OF AMPLIFYING DNA AND DNA PRODUCTS WITH INHIBITORY REGIONS**

(71) Applicant: 4basebio UK Ltd, Cambridge CB24 5QE (GB)
(72) Inventor: LANCKRIET, Heikki, Cambridge, CB24 5QE (GB); WALKER, Amy, Cambridge, CB24 5QE (GB); PAVLICKOVA, Milena, Cambridge, CB24 5QE (GB); JONES, Celia, Cambridge, CB24 5QE (GB)
(74) Representative: Boult Wade Tennant LLP

(57) **Abstract**

The present invention relates to methods of amplifying DNA comprising: a) providing a DNA template molecule comprising at least one inhibitory region; and b) amplifying the DNA template molecule to generate an amplified DNA product, wherein the DNA template molecule is amplified in the presence of at least one type of single-stranded nucleic acid (ssNA) molecule, which cannot be extended by a polymerase, and wherein the at least one type of ssNA molecule anneals to the at least one inhibitory region and improves the amplification. The ssNA molecule may be random oligonucleotides and the inhibitory regions may be a homopolymeric region, e.g. polyA, a region with a high GC content, a repeated sequence; or a sequence that is prone to forming an inhibitory structure. The invention also relates to adaptor-ligated DNA products that may be produced by the methods.

## Description

### TECHNICAL FIELD

The present invention relates to methods of amplifying DNA comprising: a) providing a DNA template molecule comprising at least one inhibitory region; and b) amplifying the DNA template molecule to generate an amplified DNA product, wherein the DNA template molecule is amplified in the presence of at least one type of single-stranded nucleic acid (ssNA) molecule, and wherein the at least one type of ssNA molecule anneals to the at least one inhibitory region. The invention also relates to adaptor-ligated DNA products that may be produced by the methods.

### BACKGROUND

When amplifying DNA, a double-stranded DNA (dsDNA) template may first be converted into single-stranded DNA (ssDNA) through a denaturation step. ssDNA is the template that enables both priming and DNA amplification machinery to bind and is therefore one of the key limiting factors in DNA amplification origin formation. However, ssDNA is naturally unstable and partially reverses back into dsDNA (renaturation) as the DNA amplification reaction progresses.

Renaturation of ssDNA can take place in form of ssDNA self-annealing which can form small loop domains or non-B DNA structures such as G-quadruplexes, Z-DNA or H-DNA structures which are highly stable, inaccessible and can be difficult to melt by DNA polymerase strand displacement. These renatured regions can result in the formation of incomplete products during DNA amplification, low product yields, or failure to amplify template ssDNA. The impact of renatured regions has been observed when amplifying large constructs (at least 8kb target region), GC-rich constructs (e.g. constructs including CAG promoter or silencer regions), constructs with repeated sequences (e.g. tandem repeats), constructs with homopolymer regions (e.g. in vitro transcription templates with 90nt+ polyadenine tail) and other high complexity regions.

Thus, a need exists for expanding the size and complexity of sequences suitable for DNA amplification, improving target product yield, as well as improving reproducibility and quality when handling high-complexity sequences.

### DESCRIPTION

The inventors of the present application have surprisingly discovered that complete and/or faithful amplification of problematic DNA template molecules can be achieved when amplification is performed in the presence of at least one type of single-stranded nucleic acid (ssNA) molecule that anneals to a problematic (or inhibitory) region of the DNA template.

The invention thus provides a method of amplifying DNA comprising: a) providing a DNA template molecule comprising at least one inhibitory region; and b) amplifying the DNA template molecule to generate an amplified DNA product, wherein the DNA template molecule is amplified in the presence of at least one type of single-stranded nucleic acid (ssNA) molecule, and wherein the at least one type of ssNA molecule anneals to the at least one inhibitory region.

The invention provides a method of amplifying DNA comprising:
a) providing a DNA template molecule comprising at least one inhibitory region; and
b) amplifying the DNA template molecule to generate an amplified DNA product, wherein the DNA template molecule is amplified in the presence of at least one type of single-stranded nucleic acid (ssNA) molecule, wherein the at least one type of ssNA molecule anneals to the at least one inhibitory region, and wherein in the absence of the at least one type of ssNA molecule the inhibitory region inhibits complete and/or faithful amplification of the DNA template molecule.

The invention provides a method of amplifying DNA comprising:
a) providing a DNA template molecule comprising at least one inhibitory region; and
b) amplifying the DNA template molecule to generate an amplified DNA product, wherein the DNA template molecule is amplified in the presence of at least one type of single-stranded nucleic acid (ssNA) molecule.

The invention provides a method of amplifying DNA comprising:
a) providing a DNA template molecule comprising at least one inhibitory region; and
b) amplifying the DNA template molecule to generate an amplified DNA product, wherein the DNA template molecule is amplified in the presence of at least one type of single-stranded nucleic acid (ssNA) molecule that cannot be extended by a polymerase.

The invention provides a method of amplifying DNA comprising:
a) providing a DNA template molecule comprising at least one inhibitory region; and
b) amplifying the DNA template molecule to generate an amplified DNA product, wherein the DNA template molecule is amplified in the presence of at least one type of single-stranded nucleic acid (ssNA) molecule, wherein the at least one type of ssNA molecule anneals to the at least one inhibitory region.

The invention provides a method of amplifying DNA comprising:
a) providing a DNA template molecule comprising at least one inhibitory region; and
b) amplifying the DNA template molecule to generate an amplified DNA product, wherein the DNA template molecule is amplified in the presence of at least one type of single-stranded nucleic acid (ssNA) molecule that cannot be extended by a polymerase, wherein the at least one type of ssNA molecule anneals to the at least one inhibitory region, and wherein in the absence of the at least one type of ssNA molecule the inhibitory region inhibits complete and/or faithful amplification of the DNA template molecule.

The invention provides a method of reducing the formation of an inhibitory structure in or involving a DNA template molecule comprising:
a) providing the DNA template molecule; and
b) incubating the DNA template molecule with at least one type of ssNA molecule which anneals to the DNA template molecule to reduce the formation of the inhibitory structure.

The invention provides a method of reducing the formation of a structure formed by self-annealing of a DNA template molecule comprising:
a) providing a DNA template molecule; and
b) incubating the DNA template molecule with at least one type of ssNA molecule which anneals to the DNA template molecule to reduce the formation of the structure formed by self-annealing of the DNA template molecule.

The invention provides a method of reducing renaturation of a DNA template molecule comprising:
a) providing a DNA template molecule; and
b) incubating the DNA template molecule with a collection of single stranded nucleic acid (ssNA) molecules which cannot be extended by a polymerase, wherein at least one type of ssNA molecule in the collection of ssNA molecules binds to the DNA template molecule to reduce the renaturation of the DNA template molecule.

The term "stabilisers" as used herein refers to the ssNA molecules (or short oligonucleotides) that are used to recover complete and/or faithful amplification which is compromised by inhibitory regions in DNA template molecules. In the absence of stabilisers (or ssNA molecules), an inhibitory region in a DNA template molecule inhibits complete and/or faithful amplification of the DNA template molecule.

The ssNA molecule(s) anneal(s) to the inhibitory region(s). The ssNA molecule(s) anneal(s) may be complementary to at least a portion of the inhibitory region(s). The ssNA molecule(s) may be at least 50%, 60%, 70%, 80%, 90% or 100% complementary to the inhibitory region or portion thereof. Thus, the ssNA molecule(s) may have one or more mismatched nucleotides relative to the inhibitory region. Such mismatches are permitted provided that the ssNA molecule(s) anneal to the inhibitory region. If the inhibitory region comprises a homopolymeric region consisting of A, T, C or G, the ssNA molecule(s) may comprise a complementary homopolymeric region consisting of T, A, G or C respectively.

In the case of a double stranded DNA template comprising a first and second strand, the inhibitory region may be in the first and/or second strand. If the inhibitory region is in the first strand, at least one type of ssNA molecule that anneals to the inhibitory region in the first strand may be used. If the inhibitory region is in the first and second strands, at least one type of ssNA molecule that anneals to the inhibitory region in the first strand and at least one type of ssNA molecule that anneals to the inhibitory region in the second strand may be used.

The DNA template molecule may be amplified in the presence of a collection of ssNA molecules that cannot be extended by a polymerase, wherein the collection of ssNA molecules comprises multiple types of ssNA molecule.

The DNA template molecule may be amplified in the presence of at least one type of ssNA molecule or in the presence of a collection of ssNA molecules comprising multiple types of ssNA molecule (including the at least one type of ssNA molecule). Each ssNA molecule of the same type has the same sequence. Each type of ssNA molecule in the collection of ssNA molecules has a different sequence to the other type(s) of ssNA molecule(s) in the collection of ssNA molecules. The collection of ssNA molecules may comprise at least 2, 3, 4, 5, 6, 7, 8, 9, 10, 11, 12, 20, 30, 40, 50, 60, 70, 80, 90 or 100 types of ssNA molecule. Preferably, the collection of ssNA molecules comprises at least 3 types of ssNA molecule. More preferably, the collection of ssNA molecules comprises at least 6 types of ssNA molecule. The collection of ssNA molecules may comprise between 10 and 80 types of ssNA molecule, between 20 and 70 types of ssNA molecule or between 30 and 60 types of ssNA molecule. Preferably, the collection comprises between 30 and 60 types of ssNA molecule. In the case of a collection of ssNAs with random sequences, the number of types of ssNA molecule and their sequences may not be known. The collection of ssNA molecules may thus comprise a plurality of types of ssNA molecule each type having a different random sequence to each other type.

Each type of ssNA molecule in the collection of ssNA molecules may have a random sequence. Random sequence may refer to a nucleotide sequence where any one of the four nucleotides (i.e., A, T, G, and C) may be present at any position in the nucleotide sequence. Each type of ssNA molecule in the collection of ssNA molecules may be a random hexamer having a random sequence of NNNNNN where "N" may be any of A, T, G, and C. Each type of ssNA molecule in the collection of ssNA molecules may have a random sequence with a terminal 3' modification that cannot be extended by a polymerase (e.g. a 3' dideoxy modified nucleotide).

The random sequence may be between 5 and 24 nucleotides in length, between 5 and 19 nucleotides in length, between 5 and 14 nucleotides in length, between 5 and 9 nucleotides in length, between 5 and 7 nucleotides in length. Preferably, the random sequence is 6 nucleotides in length. Thus, the ssNA molecule(s) may be 7 nucleotides in length including random sequence and the terminal 3' modification.

The terminal 3' modification in the collection of ssNA molecules may be the same or different between different types of ssNA molecule in the collection of ssNA molecules. The terminal 3' modification may be selected from dideoxy adenine (ddA), dideoxy thymine (ddT), dideoxy cytosine (ddC), and dideoxy guanine (ddG). The ssNA molecules in the collection of ssNA molecules may all comprise ddA, ddT, ddC or ddG.

Each ssNA molecule may have a sequence of Nₓ, wherein each N is independently selected from A, T, G, and C, and wherein x is 6-24 (preferably 6). Each ssNA molecule may have a sequence of N×N*, wherein each N is independently selected from A, T, G, and C, x is 5-24 (preferably 6) and wherein N* is a terminal 3' modification that cannot be extended by a polymerase. Each ssNA molecule may have a sequence of NₓddN, wherein each N is independently selected from A, T, G, and C, x is 5-24 (preferably 6) and wherein ddN is selected from ddA, ddT, ddC or ddG. Each N may be the same. ddN may be the same or different to N. The ssNA molecule may consist of a single type of nucleotide selected from A, T, G, and C.

The ssNA molecules may not anneal to coding DNA. The ssNA molecules may not anneal to a sequence encoding a polypeptide. The ssNA molecules may anneal to non-coding DNA. The ssNA molecules may not anneal to ribosomal ribonucleic acid (rRNA). The inhibitory region may not encode a polypeptide or portion thereof. The inhibitory region may be non-coding.

The ssNA molecules may be between 6 and 25 nucleotides in length, between 6 and 20 nucleotides in length, between 6 and 15 nucleotides in length, between 6 and 10 nucleotides in length, between 6 and 8 nucleotides in length. Preferably, the ssNA molecules are between 6 and 8 nucleotides in length. More preferably, the ssNA molecules are 7 nucleotides in length. In each case, each ssNA molecule may comprise a terminal 3' modification that cannot be extended by a polymerase (e.g. a 3' dideoxy modified nucleotide).

In the absence of the at least one type of ssNA molecule the at least one inhibitory region may:
i) result in the amplified DNA product having a different sequence to the DNA template molecule, optionally wherein the amplified DNA product comprises an insertion and/or a deletion relative to the DNA template molecule; and/or
ii) reduce the yield of the amplified DNA product.

Amplification may be performed using: a primer specific for the DNA template molecule; a pair of primers specific for the DNA template molecule; or in the presence of a primase (such as *Thermus thermophilus* (*Tth*) PrimPol (*Tth*PrimPol)). Primers specific for the DNA template molecule include single-stranded nucleic acids that are complementary to a target sequence in the DNA template molecule. A first primer of a set of primers may be complementary to the beginning of a target sequence in the DNA template molecule and a second primer of the set of primers may be complementary to the end of the target sequence in the DNA template molecule. In the case of a double-stranded DNA template, a first primer may be complementary to a first strand and a second primer may be complementary to a second strand.

Amplification may comprise rolling circle amplification (RCA), Multiple Annealing and Looping Based Amplification Cycles (MALBAC), traditional polymerase chain reaction (PCR), nucleic acid sequence-based amplification (NASBA), loop-mediated isothermal amplification (LAMP), helicase-dependent amplification (HDA), multiple displacement amplification (MDA) and recombinase polymerase amplification (RPA). Preferably, amplification comprises isothermal amplification. More preferably, amplification comprises rolling circle amplification.

Rolling circle amplification may be performed without any primers (i.e. in the presence of a primase), or in the presence of a primer or multiple primers. For example, the primer may be a synthetic primer. The primers may be primers specific for a target sequence and/or random primers. Rolling circle amplification may be performed in the presence of a primase. The primase may be *Tth*PrimPol. Preferably, if the rolling circle amplification is performed without any primers, it is performed in the presence of a primase, such as *Tth*PrimPol. If the rolling circle amplification is performed with a primase, it may also be performed in the presence of one or more primers (such as synthetic primers). Thus, rolling circle amplification may be performed with primers and with a primase. Any suitable polymerase may be used including Phi29 DNA polymerase and mutants thereof which retain functionality (e.g. QualiPhi^{®}, a chimeric form of Phi29 DNA polymerase from 4basebio). Amplification may be performed in the presence of protected nucleotide triphosphates, such as phosphorothioated nucleotide triphosphates. The incorporation of protected nucleotide triphosphates may render the amplified DNA product resistant to exonuclease digestion.

The DNA template molecule may comprise a cassette. The cassette may comprise a coding sequence. The coding sequence may encode a gene of interest, for example a gene encoding a protein. For protein expression, the cassette comprising a coding sequence may be ligated into a vector or a plasmid comprising at least a portion of a promoter so that the portion of the promoter is operably linked to the coding sequence (e.g. the promoter may be upstream of the coding sequence). The vector or plasmid may further comprise a guide RNA (gRNA) of the Clustered Regularly Interspaced Short Palindromic Repeats (CRISPR) system. The cassette may comprise at least a portion of a promoter and a coding sequence. The cassette may comprise a promoter and a coding sequence. The cassette may comprise a promoter, a coding sequence, a ribosomal binding site and a translational termination sequence. The cassette may additionally comprise sequences aiding protein expression, such as a cap-independent translation element. The cassette may comprise (or encode) a repair template (or editing template). The repair template (or editing template) may be for use in CRISPR-Cas mediated homology directed repair (HDR). The cassette may encode CRISPR guide RNA. The cassette may be a mammalian expression cassette. The promoter may be a CMV promoter. The cassette may further comprise an enhancer. The cassette may further comprise a reporter gene, such as an eGFP reporter gene or a luciferase reporter gene. The cassette may further comprise a homopolymeric sequence, such as a polyA, poly C, polyT or polyG sequence. The homopolymeric sequence may be between 3-200 nucleotides in length. The homopolymeric sequence may be used to facilitate purification of the cassette, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, 20, 30, 40, 50, 60, 70, 80, 90, 100, 110, 120, 130, 140, 150, 160, 170, 180, 190, or 200 nucleotides in length. Preferably, the homopolymeric sequence is at least 100 nucleotides in length. More preferably still, the homopolymeric sequence is at least 120 nucleotides in length. For example, the homopolymeric sequence may comprise a polyA sequence of at least 120 nucleotides. The cassette may comprise the at least one inhibitory region.

The amplified DNA product may be concatemeric, for example when amplification comprises rolling circle amplification. Thus, the amplified DNA product may comprise a plurality of cassettes (i.e. multiple copies of the same cassette). The amplified DNA product may be a concatemer comprising multiple copies of the DNA template molecule sequence.

The amplified DNA product may be digested by an endonuclease to generate digested DNA products each of which comprise a single cassette. The term "single cassette" as used herein is intended to encompass a product that does not comprise or consist of a plurality of cassettes. That is to say that the digested product comprises only a single cassette, which may comprise a single coding sequence of a gene of interest. The single cassette may not comprise or consist of a plurality of tandem repeat sequences, and/or concatemeric DNA. The term "single cassette" as used herein is intended to encompass a single copy of the DNA sequence of interest, for example, a single copy of the coding sequence. Thus, the "single cassette" may not encompass a cassette that comprises or consists of multiple copies of the same DNA sequence linked in series. The single cassette may comprise a collection of genes of interest. For example, the single cassette may comprise the sequence for at least two, three, four, or five genes of interest. The genes of interest may not be the same in a single cassette.

The methods may further comprise digesting the amplified DNA product with an endonuclease to generate digested DNA products. The amplified DNA product may comprise at least one cleavable sequence. The cleavable sequence may be an endonuclease target sequence. Thus, the amplified DNA product may comprise at least one endonuclease target sequence. Preferably, the amplified DNA molecule comprises at least two endonuclease target sequences. The endonuclease target sequences may be the same or different. Preferably, the at least one endonuclease target sequence is a restriction endonuclease target sequence. Different restriction endonuclease target sequences are known to the skilled person. For example, the restriction endonuclease target sequence may be a Bsal, Ndel, Smal, Nsil and/or Xbal target sequence. The at least one cleavable sequence (e.g. endonuclease target sequence) may be a native cleavable sequence (i.e. a cleavable sequence present in the DNA template molecule). Alternatively, the at least one cleavable sequence (e.g. endonuclease target sequence) may be introduced to the DNA template molecule prior to amplification.

After cleavage, the digested DNA products may have low dispersity (i.e. be substantially monodisperse). As used herein, the terms "low dispersity" and "monodisperse" are intended to encompass a collection of copies of digested DNA product of substantially the same size, i.e. the same length of the polynucleotide chain. That is to say, each digested DNA product may vary in size (i.e. number of bases in the polynucleotide chain) from the other digested DNA products in the library by less than 10%, preferably by less than 5%. This is in contrast to a library of polydisperse DNA products, which refers to a collection of copies of the DNA product that have an inconsistent size, i.e. an inconsistent length of the polynucleotide chain.

The methods may further comprise appending a first adaptor molecule to a first end of the digested DNA product and appending a second adaptor molecule to a second end of the digested DNA product.

Inhibitory regions may be identified by observing non-faithful and/or incomplete amplification. Such observations may be made by any means known in the art including nucleic acid electrophoresis and nucleic acid sequencing.

The at least one inhibitory region may comprise:
i) a GC content of at least 60%;
ii) a repeated sequence;
iii) a homopolymeric sequence; and/or
iv) a sequence that is prone to forming an inhibitory structure.

The at least one inhibitory region may comprise a GC content of at least 50%, at least 60%, at least 65%, at least 70%, at least 75%, at least 80%, at least 85%, at least 90%, at least 95%, at least 100%. Preferably, the at least one inhibitory region may have a GC content of at least 60%. More preferably, the at least one inhibitory region may have a GC content of at least 65%. GC content may refer to the percentage of bases that are either guanine or cytosine.

The at least one inhibitory region may comprise a repeated sequence. Repeated sequence may refer to a sequence in which two or more identical or extremely similar nucleotide sequences are repeated. A repeated sequence includes but is not limited to a tandem repeat or interspersed repeat. The repeated sequence may be a direct repeat or inverted repeat. Tandem repeats are repeated sequences which are directly adjacent to each other. Interspersed repeats are repeated sequences which are found in different locations. Direct repeats occur when a nucleotide sequence is repeated with the same directionality. Inverted repeats occur when a nucleotide sequence is repeated in the inverse direction. When there are no nucleotides separating the inverted repeat, the sequence is called a palindromic repeat. The repeated sequence may be at least 2, 5, 10, 25, 50, 75, 100, 250, 500 or 750 nucleotides long. The repeated sequence may be 2-1000 nucleotides long, 2-500 nucleotides long, 2-250 nucleotides long, 2-100 nucleotides long, 5-1000 nucleotides long, 5-500 nucleotides long, 5-250 nucleotides long, 5-100 nucleotides long. Preferably, the repeated sequence is 2-1000 nucleotides long.

The at least one inhibitory region may comprise a homopolymeric sequence. Homopolymeric sequence may refer to a sequence in which there are at least 4 repeats of the same base. Preferably, a homopolymeric sequence comprises at least 8 repeats of the same base.

If the at least one inhibitory region comprises a homopolymeric region consisting of A, T, C or G, the ssNA molecule(s) may comprise a complementary homopolymeric region consisting of T, A, G or C respectively. The at least one inhibitory region may comprise a polyA sequence and thus the ssNA molecule(s) may comprise a polyT sequence. The at least one inhibitory region may comprise a polyT sequence and thus the ssNA molecule(s) may comprise a polyA sequence. The at least one inhibitory region may comprise a polyG sequence and thus the ssNA molecule(s) may comprise a polyC sequence. The at least one inhibitory region may comprise a polyC sequence and thus the ssNA molecule(s) may comprise a polyG sequence.

The at least one inhibitory region may comprise a sequence that is prone to forming an inhibitory structure. The inhibitory structure may comprise (i) a structure that inhibits strand displacement by a polymerase; and/or (ii) a structure that inhibits a primer annealing to the DNA template molecule. The inhibitory structure may have secondary, tertiary, and/or quaternary structure. The inhibitory structure may be a non B-DNA structure. The inhibitory structure may comprise A-DNA, Z-DNA, H-DNA, a G-tetraplex, and/or a DNA triplex. The inhibitory structure(s) may be a DNA loop, cruciform, hairpin structure, and/or G-quadruplex.

Inhibitory regions may comprise a CAG promoter sequence; an insulator; a homopolymeric sequence and/or an inverted terminal repeat. Insulator may refer to a type of cis-regulatory element typically 300 bp to 2000 bp in length. An insulator may function either as an enhancer-blocker or a barrier, or both. Inhibitory regions may comprise a polyG sequence and/or an insulator.

The at least one inhibitory region may have a length of at least 4 bp, 5 bp, 6 bp, 7 bp, 8 bp. Preferably, the at least one inhibitory region has a length of at least 8 bp.

The DNA template molecule used in the methods described herein may be single-stranded or double-stranded. A single stranded DNA molecule may be generated by denaturing a double stranded DNA template molecule. Thus, the methods may further comprise denaturing a double stranded DNA template molecule to generate a single stranded DNA template molecule. Denaturation may be achieved by any means known in the art including heat and/or chemical treatment (including organic solvents such as DMSO).

The DNA template molecule may be a natural circular DNA molecule. For example, the DNA template molecule may be (i) a plasmid, (ii) a minicircle, (iii) a cosmid, or (iv) a bacterial artificial chromosome (BAC). The DNA template molecule may be an enzymatically produced circular DNA molecule. For example, the DNA template molecule may be (i) a circular DNA molecule obtained from recombinase reaction, preferably Cre recombinase reaction, or (ii) a circular DNA molecule obtained from ligase reaction, preferably using the golden gate assembly. The DNA template molecule may be an enzymatically produced covalently-closed linear DNA molecule. For example, the DNA template molecule may be (i) a DNA molecule processed with TelN protelomerase; or (ii) a DNA molecule generated by ligation of the DNA ends with an adaptor.

The DNA template molecule may be linear. If the DNA template molecule is linear, prior to amplification (e.g. rolling circle amplification), a DNA template molecule may be circularized to produce a DNA template molecule suitable for use in the methods described herein.

The DNA template molecule may comprise a cassette. The cassette may be a mammalian expression cassette. The cassette may further comprise a promoter. The promoter may be a CMV promoter. The cassette may further comprise an enhancer. The cassette may further comprise a reporter gene, such as an eGFP reporter gene or a luciferase reporter gene. The cassette may further comprise a homopolymeric sequence. The cassette may further comprise a LoxP sequence, preferably two LoxP sequences. If the two LoxP sequences are oriented in the same direction, the DNA sequence between the two LoxP sequences is excised as a circular loop of DNA. If the two LoxP sequences are oriented in the opposite direction, the DNA sequence between the two LoxP sequences is inverted. Thus, preferably, the two LoxP sequences are in the same orientation (i.e. the same direction) in the template DNA molecule.

The ssNA molecules may not be extendable by a polymerase. The ssNA molecules may not be extendable by a polymerase due to a terminal 3' modification. The terminal 3' modification may be a 3' dideoxy modified nucleotide, a 3' inverted nucleotide, a 3' C3 spacer, a 3' amino, or a 3' phosphorylation. The ssNA molecules may be locked nucleic acids (LNA) or peptide nucleic acids (PNA) and thus cannot be extended by a polymerase. Preferably the ssNA molecules are not extendable by a polymerase due to a terminal 3' modification, optionally wherein the terminal 3' modification comprises a dideoxy modified nucleotide.

The amplified DNA product may comprise double-stranded regions and single-stranded regions. Preferably, the amplified DNA product is a double-stranded DNA molecule.

The methods may further comprise:
(a) contacting the amplified DNA product with an endonuclease and first and second adaptor molecules to form a single contiguous aqueous volume; and
(b) incubating the single contiguous aqueous volume to generate an adaptor-ligated DNA product, wherein the adaptor-ligated DNA product comprises a linear double-stranded region, and wherein the first adaptor molecule is appended to a first end of the linear double-stranded region and the second adaptor molecule is appended to a second end of the linear double-stranded region.

Preferably, the step of contacting the amplified DNA product with the endonuclease, the ligase and first and second adaptor molecules is performed in a single reaction (i.e. a single step).

The first and second adaptor molecules may be identical molecules or they may be different molecules. For example, the first adaptor molecule and/or the second adaptor molecule may comprise a hairpin. The first adaptor molecule and/or the second adaptor molecule may be double-stranded linear nucleic acid molecules comprising one or more nuclease-resistant nucleotides. The first adaptor molecule may comprise a hairpin and the second adaptor molecule may be a double-stranded linear nucleic acid molecule comprising one or more nuclease-resistant nucleotides. Thus, the adaptor-ligated DNA product may be resistant to nuclease (e.g. exonuclease) digestion.

The step of contacting the amplified DNA product with the endonuclease and first and second adaptor molecules is preferably performed in the presence of a ligase. Thus, the invention provides a method for producing an adaptor-ligated DNA product, wherein the method comprises:
(a) contacting the amplified DNA product with an endonuclease, a ligase and first and second adaptor molecules to form a single contiguous aqueous volume; and
(b) incubating the single contiguous aqueous volume to generate the adaptor-ligated DNA product, wherein the adaptor-ligated DNA product comprises a linear double-stranded region, and wherein the first adaptor molecule is appended to a first end of the linear double-stranded region and the second adaptor molecule is appended to a second end of the linear double-stranded region.

The appending (or linking or closing) of the first adaptor molecule and/or the second adaptor molecule may be performed by hybridization or ligation of the adaptor molecules to the ends of the digested DNA product. The digested DNA product may comprise or consist of a linear double-stranded region. Thus, the first adaptor molecule may be hybridized to the first end of the linear double-stranded region. The second adaptor molecule may be hybridized to the second end of the linear double-stranded region. The first adaptor molecule may be ligated to the first end of the linear double-stranded region. The second adaptor molecule may be ligated to the second end of the linear double-stranded region. The appending of the first adaptor molecule and the second adaptor molecule may be performed by both hybridization and ligation of the adaptor molecules to the ends of the linear double-stranded region. Thus, the first adaptor molecule may be hybridized and ligated to the first end of the linear double-stranded region. The second adaptor molecule may be hybridized and ligated to the second end of the linear double-stranded region. The appending may be performed via a linker or spacer molecule which facilitates joining of the adaptor molecule to the first and/or second end of the linear double-stranded region.

The linker or spacer may be at least 10, at least 20, at least 30, at least 40, at least 50, at least 60, at least 70, at least 80, at least 90, at least 100, at least 125, at least 150, at least 175, or at least 200 base pairs long.

The invention provides a method for producing an adaptor-ligated DNA product, the method comprises:
(a) amplification of a DNA template molecule as described herein, wherein the DNA template molecule comprises at least one cleavable (e.g. endonuclease) target sequence to generate amplified DNA product;
(b) contacting the amplified DNA product with an endonuclease, a ligase and first and second adaptor molecules to form a single contiguous aqueous volume; and
(c) incubating the single contiguous aqueous volume to generate the adaptor-ligated DNA product, wherein the adaptor-ligated DNA product comprises a linear double-stranded region, and wherein the first adaptor molecule is appended to a first end of the linear double-stranded region and the second adaptor molecule is appended to a second end of the linear double-stranded region.

The methods may further comprise, after the step of amplification and before the step of contacting the amplified DNA product with an endonuclease, a ligase and first and second adaptor molecules, a step of heat-deactivation. Thus, the invention provides a method for producing an adaptor-ligated DNA product, the method comprises:
(a) amplification of a DNA template molecule as described herein, wherein the DNA template molecule comprises at least one cleavable (e.g. endonuclease) target sequence to generate amplified DNA product;
(b) heat-deactivation of the reaction of step (a);
(c) contacting the amplified DNA product with an endonuclease, a ligase and first and second adaptor molecules to form a single contiguous aqueous volume; and
(d) incubating the single contiguous aqueous volume to generate the adaptor-ligated DNA product, wherein the adaptor-ligated DNA product comprises a linear double-stranded region, and wherein the first adaptor molecule is appended to a first end of the linear double-stranded region and the second adaptor molecule is appended to a second end of the linear double-stranded region.

The step of heat-deactivation may be performed under conditions sufficient to inactivate the reagents used during the amplification reaction. The step of heat-deactivation may be performed at a temperature of at least 50°C, at least 55°C, at least 60°C, at least 65°C, at least 70°C, at least 75°C, at least 80°C, at least 85°C, at least 90°C, at least 95°C, or at least 100°C. The step of heat-deactivation may be performed for at least 1 min, at least 3 mins, at least 5 mins, at least 10 mins, at least 15 mins, or at least 20 mins.

In the method described herein, after the step of amplification, the step of contacting the amplified DNA product with an endonuclease, a ligase and first and second adaptor molecules to form a single contiguous aqueous volume may be performed without purifying the product of the amplification reaction. That is to say that the step of contacting the amplified DNA product with an endonuclease, a ligase and first and second adaptor molecules may be performed directly after the step of amplification. The step of contacting the amplified DNA product with an endonuclease, a ligase and first and second adaptor molecules may be performed directly after the step of heat-deactivation.

The method may further comprise, after the step of incubating the single contiguous aqueous volume, a step of purification of the adaptor-ligated DNA product.

The method may further comprise, after the step of incubating the single contiguous aqueous volume, a step of nuclease digestion. The nuclease digestion may be exonuclease digestion, such as exonuclease I and/or exonuclease III digestion. The step of nuclease digestion may take place before or after the step of purification. The step of nuclease digestion may allow for removal of any double-stranded DNA molecules and/or adaptor molecules which have not been used to produce adaptor-ligated DNA product. Thus, the method for producing an adaptor-ligated DNA product may comprise the steps:
(a) contacting the amplified DNA product with an endonuclease, a ligase and first and second adaptor molecules to form a single contiguous aqueous volume;
(b) incubating the single contiguous aqueous volume to generate the adaptor-ligated DNA product, wherein the adaptor-ligated DNA product comprises a linear double-stranded region, and wherein the first adaptor molecule is appended to a first end of the linear double-stranded region and the second adaptor molecule is appended to a second end of the linear double-stranded region; and
(c) incubating the single contiguous aqueous volume with a nuclease (e.g. an exonuclease).

The method for producing an adaptor-ligated DNA product may comprise the steps:
(a) amplification of a DNA template molecule as described herein, wherein the DNA template molecule comprises at least one cleavable (e.g. endonuclease) target sequence to generate amplified DNA product;
(b) contacting the amplified DNA product with an endonuclease, a ligase and first and second adaptor molecules to form a single contiguous aqueous volume;
(c) incubating the single contiguous aqueous volume to generate the adaptor-ligated DNA product, wherein the adaptor-ligated DNA product comprises a linear double-stranded region, and wherein the first adaptor molecule is appended to a first end of the linear double-stranded region and the second adaptor molecule is appended to a second end of the linear double-stranded region; and
(d) incubating the single contiguous aqueous volume with a nuclease (e.g. an exonuclease).

The method for producing an adaptor-ligated DNA product may comprise the steps:
(a) amplification of a DNA template molecule as described herein, wherein the DNA template molecule comprises at least one cleavable (e.g. endonuclease) target sequence;
(b) contacting the amplified DNA product with an endonuclease, a ligase and first and second adaptor molecules to form a single contiguous aqueous volume;
(c) incubating the single contiguous aqueous volume to generate the adaptor-ligated DNA product, wherein the adaptor-ligated DNA product comprises a linear double-stranded region, and wherein the first adaptor molecule is appended to a first end of the linear double-stranded region and the second adaptor molecule is appended to a second end of the linear double-stranded region;
(d) purification of the adaptor-ligated DNA product; and
(e) incubating the product of step (d) with a nuclease (e.g. an exonuclease).

The method for producing an adaptor-ligated DNA product may comprise the steps:
(a) amplification of a DNA template molecule as described herein, wherein the DNA template molecule comprises at least one cleavable (e.g. endonuclease) target sequence;
(b) contacting the amplified DNA product with an endonuclease, a ligase and first and second adaptor molecules to form a single contiguous aqueous volume;
(c) incubating the single contiguous aqueous volume to generate the adaptor-ligated DNA product, wherein the adaptor-ligated DNA product comprises a linear double-stranded region, and wherein the first adaptor molecule is appended to a first end of the linear double-stranded region and the second adaptor molecule is appended to a second end of the linear double-stranded region;
(d) incubating the single contiguous aqueous volume with a nuclease (e.g. an exonuclease); and
(e) purification of the DNA product.

The endonuclease may be a restriction endonuclease (such as a Type II restriction endonuclease), RNA-guided DNA endonuclease, meganuclease or homing endonuclease. The Type II restriction endonuclease may be a Type IIP restriction endonuclease (e.g. EcoRl, Hindlll, BamHI or Notl) or a Type IIS restriction endonuclease (e.g. Bsal, Bbvl, Fokl or Sapl). The RNA-guided DNA endonuclease may be an endonuclease of Class 2 e.g. Class 2 Type V. The RNA-guided DNA endonuclease may be Cas12a. The RNA-guided DNA endonuclease may be Cpf1 or Mad7. Preferably, the RNA-guided DNA endonuclease is Cpf1. The homing endonuclease may be I-Ceul, I-Scel, PI-Pspl or PI-Scel.

The endonuclease may be a restriction enzyme endonuclease. The endonuclease may be a Type IIS restriction enzyme. The endonuclease may be any enzyme that recognizes a DNA sequence and cleaves outside of the recognition sequence. For example, the endonuclease may be a Bbsl, Bsal, BsmBI, BspQl, BtgZl, Esp3I,SapI, Aarl, Acc36I, AcIWI, Acul, Ajul, Alol, Alw26I, Alwl, Arsl, AsuHPI, Bael, Barl, Bbvl, Bccl, BceAl, Bcgl, BciVI, BcoDI, BfuAl, Bful, Bmrl, Bmsl, Bmul, Bpil, Bpml, BpuEl, BsaXI, Bse1I, Bse3DI, BseGI, BseMl, BseMll, BseNI, BseRl, BseXl, Bsgl, BsIFI, BsmAl, BsmFl, Bsml, Bso31I, BspCNI, BspMI, BspPl, BspQl, BspTNI, BsrDl, Bsrl, Bst6I, BstF5I, BstMAI, BstV1I, BstV2I, Bsul, BtgZl, BtsCl, Btsl-v2, BtsMutl, Bvel, Csel, CspCI, Eam1104I, Earl, Ecil, Eco31I, Eco57I, Esp3I, Faql, Faul, Fokl, Gsul, Hgal, Hphl, HpyAV, Lgul, Lmnl, Lsp1109I, Lwel, Mboll, Mlyl, Mmel, Mnll, Mva1269I, NmeAIII, PaqCl, PciSI, Pctl, PleI, Ppsl, Psrl, Schl, SfaNI, Taqll, TspDTI and/or TspGWI restriction enzyme.

Type IIS restriction endonucleases cleave the amplified DNA molecule outside of the recognition sequence (i.e. an endonuclease target sequence), which means that the recognition sequence (i.e. endonuclease target sequence) is not included in the adaptor-ligated DNA product.

The step of incubating the single contiguous aqueous volume may be performed under conditions that promote appending (or linking) of the first and second adaptor molecules to the digested DNA product. The appending may be performed by creating a covalent link between the first and/or second adaptor molecule and the first and/or second end of the digested DNA product.

The step of incubating the single contiguous aqueous volume may be performed under conditions that promote digestion of the amplified DNA product to produce the digested DNA product. The digestion of the amplified DNA product to produce the digested DNA product may be performed at a first temperature of 1°C-100°C, 1°C -80°C, 5°C-70°C, 10°C-60°C, 15°C-55°C, 20°C-50°C, 25°C -45°C, 30°C-40°C, 35°C-39°C, 36°C-38°C, or at about 37°C. The digestion may be endonuclease digestion, preferably Type IIS endonuclease digestion.

The step of incubating the single contiguous aqueous volume may be performed under conditions that promote ligation of the digested DNA products to the first and second adaptor molecules. The ligation may be at least 5%, at least 10%, at least 15, at least 20%, at least 25%, at least 30%, at least 35%, at least 40, at least 45%, at least 50%, at least 55%, at least 60, at least 65, at least 70%, at least 75, at least 80%, at least 82%, at least 85%, at least 90%, or at least 95% efficient. For example, at least 5%, at least 10%, at least 15, at least 20%, at least 25%, at least 30%, at least 35%, at least 40, at least 45%, at least 50%, at least 55%, at least 60, at least 65, at least 70%, at least 75, at least 80%, at least 82%, at least 85%, at least 90%, or at least 95% of the digested DNA products may be incorporated into adaptor-ligated DNA products. Preferably, the ligation is at least 15% efficient.

Ligation efficiency may be established based on DNA quantification values before and after the digestion/ligation reaction. Thus, ligation efficiency may be established based on the equation: (starting amplified DNA amount) / (final linear DNA amount) × 100%.

Ligation efficiency may also be established based on DNA quantification values before and after the digestion/ligation reaction and the subsequent exonuclease treatment to remove remaining DNA constructs and adaptor molecules excess.

For example, the amplified DNA molecule generated by amplification (e.g. rolling circle amplification) may first be quantified so that the amount of the starting material during the digestion/ligation reaction is known. After all the enzymatic reactions, the adaptor-ligated DNA product may be quantified to calculate the ligation efficiency as per the equation above.

DNA quantification methods are known to a person skilled in the art. For example, DNA quantifications may be carried out using the Qubit dsDNA BR assay from ThermoFisher (https://www.thermofisher.com/order/catalog/product/Q32850#/Q32850).

The step of ligation of the digested DNA product to the first and second adaptor molecules may be performed at a second temperature of 1°C -90°C, 2°C -70°C, 5°C-60°C, 8°C-55°C, 9°C-50°C, 10°C-45°C, 11°C-40°C, 12°C-37°C, 13°C-30°C, 14°C-25°C, 15°C-20°C or at about 16°C.

The step of incubating the single contiguous aqueous volume may comprise incubating at a first temperature and then incubating at a second temperature. The first temperature may be 1°C-100°C, 1°C-80°C, 5°C-70°C, 10°C-60°C, 15°C-55°C, 20°C-50°C, 25°C-45°C, 30°C-40°C, 35°C-39°C, 36°C-38°C, or about 37°C. The second temperature may be 1°C -90°C, 2°C -70°C, 5°C-60°C, 8°C-55°C, 9°C-50°C, 10°C-45°C, 11°C-40°C, 12°C-37°C, 13°C-30°C, 14°C-25°C, 15°C-20°C or at about 16°C. Preferably, the first temperature is 35°C-39°C and the second temperature is 14°C-18°C. Using these conditions the endonuclease may be a Type IIS restriction endonuclease (e.g. Bsal) and the ligase may be T4 DNA ligase, T7 DNA ligase, mammalian DNA ligase I, III and IV; Taq DNA ligase, Tth DNA ligase, or E. *coli* DNA ligase.

The step of incubating the single contiguous aqueous volume may be performed isothermally. The step of incubating the single contiguous aqueous volume may comprise incubating at a constant temperature. The constant temperature promotes simultaneous digestion of the amplified DNA product to produce the digested DNA product and ligation of the digested DNA product to the first and second adaptor molecules. For example, the constant temperature may be 20°C, 21°C, 22°C, 23°C, 24°C, 25°C, 26°C, 27°C, 28°C, 29°C, 30°C, 31°C, 32°C, 33°C, 34°C, 35°C, 36°C, 37°C, 38°C, 39°C, or 40°C. Preferably, the constant temperature is 30°C. The constant temperature is intended to mean that the temperature does not significantly change during the reaction. The constant temperature is intended to mean that the temperature variation during the step of incubating the single contiguous aqueous volume is less than 10°C, less than 9°C, less than 8°C, less than 7°C, less than 6°C, less than 5°C, less than 4°C, less than 3°C, less than 2°C, or less than 1°C. In a preferred embodiment the temperature during the step of incubating the single contiguous aqueous does not deviate by more than 5°C, preferably by not more than 3°C, even more preferably not more than 1°C. Thus, the constant temperature may be a temperature in a range of 20°C-30°C, 22°C-32°C, 24°C-34°C, 26°C-36°C, 28°C-38°C, 30°C-40°C, 22°C-28°C, 32°C-38°C, 25°C-35°C, 26°C-34°C, 27°C- 33°C, 27.5°C-32.5°C, 28°C-32°C, 28.5°C-31.5°C, 29°C-31°C, or 29.5°C-30.5°C. Preferably, the constant temperature is a temperature in a range of 27.5°C-32.5°C. Alternatively, the constant temperature may be a temperature in a range of 32°C-42°C, 33°C-41°C, 34°C-40°C, 35°C-39°C, 36°C-38°C. Preferably, the constant temperature is a temperature in a range of 34.5°C-39.5°C.

The step of incubating the single contiguous aqueous volume may comprise cycling between the first temperature and the second temperature. The step of incubating the single contiguous aqueous volume may comprise cycling between the first temperature and the second temperature at least 2, at least 3, at least 4, at least 5, at least 6, at least 7, at least 8, at least 9, at least 10, at least 15, at least 20, at least 25, at least 30, at least 35, at least 40, at least 45, at least 50, at least 55, at least 60, at least 65, at least 70, at least 80, at least 90, or at least 100 times, preferably at least 20 times. The step of incubating the single contiguous aqueous volume may comprise cycling between the first temperature and the second temperature less than 40, less than 35, less than 30 times, less than 29, less than 25 times. The step of incubating the single contiguous aqueous volume may comprise cycling between the first temperature and the second temperature 2-100, 5-80, 10-70, 20-60, or 30-60 times. The step of incubating the single contiguous aqueous volume may comprise cycling between the first temperature and the second temperature 2-20, 5-29, 61-100, or 65-80 times.

The DNA template molecule may comprise at least one cleavable target sequence. The cleavable target sequence may be an endonuclease target sequence. Thus, the DNA template molecule may comprise at least one endonuclease target sequence. Preferably, the DNA template molecule comprises at least two endonuclease target sequences. The endonuclease target sequences may be the same or different. Preferably, the at least one endonuclease target sequence is a restriction endonuclease target sequence. Different restriction endonuclease target sequences would be known to the skilled person. The cleavable target sequence may be a Type IIS restriction endonuclease target sequence. For example, the restriction endonuclease target sequence may be a Bbsl, Bsal, BsmBI, BspQl, BtgZl, Esp3I,SapI, Aarl, Acc36I, AcIWI, Acul, Ajul, Alol, Alw26I, AlwI, Arsl, AsuHPI, Bael, Barl, Bbvl, Bccl, BceAl, Bcgl, BciVI, BcoDI, BfuAl, Bful, Bmrl, Bmsl, Bmul, Bpil, Bpml, BpuEl, BsaXI, Bse1I, Bse3DI, BseGI, BseMl, BseMll, BseNI, BseRl, BseXl, Bsgl, BsIFI, BsmAl, BsmFl, Bsml, Bso31I, BspCNI, BspMI, BspPl, BspQl, BspTNI, BsrDl, Bsrl, Bst6I, BstF5I, BstMAI, BstV1I, BstV2I, Bsul, BtgZl, BtsCl, Btsl-v2, BtsMutl, Bvel, Csel, CspCI, Eam1104I, Earl, Ecil, Eco31I, Eco57I, Esp3I, Faql, Faul, Fokl, Gsul, Hgal, Hphl, HpyAV, Lgul, Lmnl, Lsp1109I, Lwel, Mboll, Mlyl, Mmel, Mnll, Mva1269I, NmeAIII, PaqCl, PciSI, Pctl, PleI, Ppsl, Psrl, Schl, SfaNI, Taqll, TspDTI and/or TspGWI target sequence. The at least one cleavable sequence (e.g. endonuclease target sequence) may be a native cleavable sequence (i.e. a cleavable sequence present in the template molecule). Alternatively, the at least one cleavable sequence (e.g. endonuclease target sequence) may be introduced to the DNA template molecule prior to the production of the adaptor-ligated DNA product.

The endonuclease may be a restriction enzyme endonuclease. The endonuclease may be a Type IIS restriction enzyme. The endonuclease may be any enzyme that recognizes a DNA sequence and cleaves outside of the recognition sequence. For example, the endonuclease may be a Bbsl, Bsal, BsmBI, BspQl, BtgZl, Esp3I,SapI, Aarl, Acc36I, AcIWI, Acul, Ajul, Alol, Alw26I, AlwI, Arsl, AsuHPI, Bael, Barl, Bbvl, Bccl, BceAl, Bcgl, BciVI, BcoDI, BfuAl, Bful, Bmrl, Bmsl, Bmul, Bpil, Bpml, BpuEl, BsaXI, Bse1I, Bse3DI, BseGI, BseMl, BseMll, BseNI, BseRl, BseXl, Bsgl, BsIFI, BsmAl, BsmFl, Bsml, Bso31I, BspCNI, BspMI, BspPl, BspQl, BspTNI, BsrDl, Bsrl, Bst6I, BstF5I, BstMAI, BstV1I, BstV2I, Bsul, BtgZl, BtsCl, Btsl-v2, BtsMutl, Bvel, Csel, CspCI, Eam1104I, Earl, Ecil, Eco31I, Eco57I, Esp3I, Faql, Faul, Fokl, Gsul, Hgal, Hphl, HpyAV, Lgul, Lmnl, Lsp1109I, Lwel, Mboll, Mlyl, Mmel, Mnll, Mva1269I, NmeAIII, PaqCl, PciSI, Pctl, PleI, Ppsl, Psrl, Schl, SfaNI, Taqll, TspDTI and/or TspGWI restriction enzyme.

The ligase may be a DNA ligase, such as a T4 DNA ligase, T7 DNA ligase, mammalian DNA ligase I, III and IV; Taq DNA ligase, Tth DNA ligase, or *E. coli* DNA ligase.

The first adaptor molecule and/or the second adaptor molecule may comprise one or more locked nucleic acids (LNAs).

The first adaptor molecule and/or the second adaptor molecule may comprise one or more protected nucleotides (i.e. nuclease-resistant nucleotides), such as phosphorothioated nucleotides.

The first and second adaptor molecules may comprise one or more phosphorothioated nucleotides, such that, once the adaptor molecules are appended (e.g. ligated) to the digested DNA product, the adaptor-ligated DNA product is resistant to nuclease digestion or has improved or enhanced resistance to nuclease digestion. The adaptor-ligated DNA product may be resistant to 3'-end exonuclease digestion (e.g. by exonuclease III) and/or 5'-end exonuclease digestion (e.g. by exonuclease VIII).

The adaptor molecule may comprise a plurality of phosphorothioated nucleotides. For example, the adaptor molecules may comprise at least 2, at least 3, at least 4, at least 5, at least 6, at least 7, at least 8, at least 9, at least 10, at least 11, at least 12, at least 13, at least 14, at least 15 or at least 16 phosphorothioated nucleotides in each strand.

The adaptor molecule may be a nucleic acid adaptor molecule. The adaptor molecule may be double-stranded. The adaptor molecule may comprise a portion that is double-stranded.

The first and/or second adaptor molecules may comprise at least 3, at least 4, at least 5, at least 6, at least 7, at least 8, at least 9, at least 10, at least 11, at least 12, at least 13, at least 14, at least 15, or at least 16 base pairs.

The adaptor molecule may comprise a plurality of phosphorothioated nucleotides in each strand. For example, the adaptor molecules may comprise at least 2, at least 3, at least 4, at least 5, at least 6, at least 7, at least 8, at least 9, at least 10, at least 11, at least 12, at least 13, at least 14, at least 15 or at least 16 phosphorothioated nucleotides in each strand.

The adaptor molecule may comprise a plurality of phosphorothioated nucleotides at internal positions in each strand. For example, the adaptor molecules may comprise at least 1, at least 2, at least 3, at least 4, at least 5, at least 6, at least 7, at least 8, at least 9, at least 10, at least 11, at least 12, at least 13, at least 14, at least 15 or at least 16 phosphorothioated nucleotides at internal positions in each strand. Preferably, the adaptor molecule comprises at least 2 phosphorothioated nucleotides at internal positions in each strand.

The internal positions may not be located between the second and penultimate nucleotide of the adaptor molecule. The internal positions may be any position in the adaptor molecules other than the last nucleotide at the end of each strand.

The adaptor molecule may comprise at least 5%, at least 10%, at least 20%, at least 30%, at least 40%, at least 50%, at least 60%, at least 70%, at least 80%, at least 90%, at least 95%, or at least 100% of protected nucleotides.

The nucleotides resistant to exonuclease digestion (i.e. protected nucleotides) may be phosphorothioated nucleotides of at least one type. For example, the at least one type of phosphorothioated nucleotides is α-S-dATP (i.e. 2'-deoxyadenosine-5'-(α-thio)-triphosphate), α-S-dCTP (i.e. 2'-deoxycytidine-5'-(α-thio)-triphosphate), α-S-dGTP (i.e. 2'-deoxyguanosine-5'-(α-thio)-triphosphate), α-S-dTTP (i.e. 2'-deoxythymidine-5'-(α-thio)-triphosphate), α-S-dUTP (i.e. 2'-deoxyuridine-5'-(α-thio)-triphosphate), and/or uridine 2', 3'-cyclophosphorothioate.

The adaptor molecules may comprise at least two types of phosphorothioated nucleotides. For example, the at least two types of phosphorothioated nucleotides are: α-S-dATP and α-S-dCTP, α-S-dATP and α-S-dGTP, α-S-dATP and α-S-dTTP, α-S-dCTP and α-S-dGTP, α-S-dCTP and α-S-dTTP, or α-S-dGTP and α-S-dTTP.

The adaptor molecules may comprise at least three types of phosphorothioated nucleotides. For example, the at least three types of phosphorothioated nucleotides are:
a. α-S-dATP, α-S-dCTP and α-S-dGTP;
b. α-S-dATP, α-S-dCTP and α-S-dTTP;
c. α-S-dATP, α-S-dGTP and α-S-dTTP; or
d. α-S-dCTP, α-S-dGTP and α-S-dTTP.

The adaptor molecules may comprise at least four types of phosphorothioated nucleotides. For example, the at least four types of protected nucleotides are α-S-dATP, α-S-dCTP, α-S-dGTP and α-S-dTTP.

The phosphorothioated nucleotides may be Sp-isomers, Rp-isomers or a mixture of both Sp- and Rp-isomers.

The nucleotides resistant to exonuclease digestion (i.e. protected nucleotides) may be MOE nucleotides of at least one type, or at least two, three or four types. For example, the MOE nucleotides may be 2'-O-methoxy-ethyl guanosine, 2'-O-methoxy-ethyl cytidine, 2'-O-methoxy-ethyl adenosine, and/or 2'-O-methoxy-ethyl thymidine.

The first end of the digested DNA product may be complementary to a portion of the first adaptor molecule. The second end of the digested DNA product may be complementary to a portion of the second adaptor molecule. The first end and/or the second end of the digested DNA product may be generated by endonuclease digestion as described herein.

As used herein, the term "complementary" refers to the pairing of nucleotide sequences according to Watson/Crick pairing rules. For example, a sequence 5'-GCGGTCCCA-3' has the complementary sequence of 5'-TGGGACCGC-3'. A complement sequence can also be a sequence of RNA complementary to the DNA sequence.

The first adaptor molecule and/or the second adaptor molecule may comprise a functional portion. The functional portion may be a binding molecule, a targeting sequence, or a probe.

The functional portion may be a probe. As used herein, the term "probe" refers to a fragment of DNA, RNA or DNA/RNA chimera of variable length (e.g. 3-1000 bases long), which is used to detect the presence of target nucleotide sequences that are complementary to the sequence in the probe. Typically, the probe hybridizes to single-stranded nucleic acid whose base sequence allows probe-target base pairing due to complementarity between the probe and target. Thus, the functional portion may be a DNA sequence, a RNA sequence or a DNA/RNA chimera sequence. As used herein, the term "complementary" refers to the pairing of nucleotide sequences according to Watson/Crick pairing rules. For example, a sequence 5'-GCGGTCCCA-3' has the complementary sequence of 5'-TGGGACCGC-3'. A complement sequence can also be a sequence of RNA complementary to the DNA sequence.

The functional portion may be a binding molecule. The term "binding molecule" refers to any molecule capable of binding to the adaptor-ligated DNA product described herein and/or that is capable of binding to a further molecule or target. The binding molecule may be a protein, a polypeptide, or a peptide. The binding molecule may be an antibody, such as a monoclonal antibody or a polyclonal antibody. The binding molecule may be an antibody fragment.

The functional portion may facilitate detection of the DNA product by binding to capture molecules (e.g. capture antibodies bound by protein-protein interactions). The functional portion may bind to a cell target, for example, a cell receptor.

The functional portion may be a label. The 'label' can be any chemical entity which enable the detection of the double-stranded nucleic acid molecule via, physical, chemical and/or biological means. The label may be a chromophore, a fluorophore and/or a radioactive molecule.

The functional portion may be a targeting sequence. The targeting sequence may be a fragment of DNA or RNA of variable length, which is used to target the DNA product to a specific location in a cell. The targeting sequence may be used to increased transfection efficiency of non-viral gene delivery by virtue of enhanced nuclear import of the adaptor-ligated DNA product. For example, the targeting sequence may be a DNA nuclear targeting sequences (i.e. a recognition sequence for endogenous DNA-binding proteins), such as SV40 enhancer sequence (preferably downstream from the cassette).

To facilitate detection and/or quantification of the adaptor-ligated DNA product, the functional portion may comprise a fluorophore, a radioactive compound or a barcode.

The functional portion may also facilitate DNA sequencing. For example, the functional portion may be a sequencing adapter. The term "sequencing adapter" is intended to encompass one or more nucleic acid domains that include at least a portion of a nucleic acid sequence (or complement thereof) utilized by a sequencing platform of interest, such as a sequencing platform provided by Illumina^{®} (e.g. the HiSeq^{™}, MiSeq^{™} and/or Genome Analyzer^{™} sequencing systems), Oxford Nanopore^{™} Technologies (e.g. the MinION sequencing system), Ion Torrent^{™} (e.g. the Ion PGM^{™} and/or Ion Proton^{™} sequencing systems), Pacific Biosciences (e.g. the PACBIO RS II sequencing system); Life Technologies^{™} (e.g. a SOLiD sequencing system), Roche (e.g. the 454 GS FLX+ and/or GS Junior sequencing systems), or any other sequencing platform of interest.

The first adaptor molecule and/or the second adaptor molecule may comprise an inverted terminal repeat sequence. The inverted terminal repeat sequences of the first adaptor molecule and the second adaptor molecule may be symmetrical (i.e. have the same symmetrical three-dimensional organization with respect to each other) or asymmetrical (i.e. have different three-dimensional organization with respect to each other). The inverted terminal repeat sequences of the first adaptor molecule and the second adaptor molecule may be from the same or different serotypes. An inverted terminal repeat sequence may comprise a terminal resolution site and a Rep binding site.

The first adaptor molecule and/or the second adaptor molecule may comprise an aptamer.

The first adaptor molecule and/or the second adaptor molecule may confer resistance to the nuclease digestion, such as exonuclease digestion (e.g. exonuclease I and/or exonuclease III digestion).

The first adaptor molecule may be a nucleic acid adaptor molecule. The second adaptor molecule may be a nucleic acid adaptor molecule. The first adaptor molecule and/or the second adaptor molecule may comprise a self-complementary element which creates a loop, such as a hairpin loop or a stem loop. Thus, the first adaptor molecule may comprise a hairpin or a stem-loop. The second adaptor molecule may comprise a hairpin or a stem-loop. Both the first and second adaptor molecules may comprise a hairpin or a stem-loop. The adaptor molecules may each comprise a double-stranded portion comprising a sense strand and an antisense strand, wherein the sense strand and the antisense strand are linked together in a hairpin such that the sense strand is hybridized to the antisense strand. The double-stranded portion of an adaptor may comprise a 3' overhang or a 5' overhang of at least 1, at least 2, at least 3, at least 4, or at least 5 nucleotides. Preferably the 3' overhang or the 5' overhang is 4-8 nucleotides. Each end of the linear double-stranded region (or linear portion of the double-stranded DNA molecule) may comprise a 3' or a 5' overhang. A portion of the first adaptor molecule (e.g. the overhang) may be complementary to the first end of the linear double-stranded region. A portion of the second adaptor molecule may be complementary to the second end of the linear double-stranded region.

The adaptor-ligated DNA product may be a closed linear DNA product, optionally a covalently closed linear DNA product. Thus, in embodiments where the adaptor molecules comprise a loop (e.g. a hairpin), the adaptor molecules close the ends of the linear double-stranded region forming a covalently closed linear DNA product. When only one of the first or second adaptor molecules comprises a loop, a partially closed linear DNA molecule will result.

The adaptor-ligated DNA product may be a linear double-stranded DNA molecule, a closed linear DNA molecule or a partially closed linear DNA molecule. A linear double-stranded DNA molecule may result from the first and second adaptor molecules being linear double-stranded DNA molecules. A closed linear DNA molecule may result from the first and second adaptor molecules comprising a hairpin or stem-loop. A partially closed linear DNA molecule may result from one of the first or second adaptor molecules being a linear double-stranded DNA molecule and other comprising a hairpin or stem-loop. Linear double-stranded adaptors may comprise protected nucleotides as described herein thereby conferring exonuclease resistance on the adaptor-ligated DNA product. Methods for producing closed linear DNA products, linear double-stranded DNA products and partially closed linear DNA products are provided in WO2023/006978A1, which is incorporated herein by reference.

The invention provides an adaptor-ligated DNA product comprising a linear double-stranded region, a first adaptor molecule and a second adaptor molecule, wherein the first adaptor molecule is appended to a first end of the linear double-stranded region and the second adaptor molecule is appended to a second end of the linear double-stranded region. The linear double-stranded region may comprise an inhibitory region.

The inhibitory region may comprise:
i) a GC content of at least 60%;
ii) a repeated sequence;
iii) a homopolymeric sequence; and/or
iv) a sequence that is prone to forming an inhibitory structure.

The at least one inhibitory region may comprise a GC content of at least 50%, at least 60%, at least 65%, at least 70%, at least 75%, at least 80%, at least 85%, at least 90%, at least 95%, at least 100%. Preferably, the at least one inhibitory region may have a GC content of at least 60%. More preferably, the at least one inhibitory region may have a GC content of at least 65%. GC content may refer to the percentage of bases that are either guanine or cytosine.

The at least one inhibitory region may comprise a repeated sequence. Repeated sequence may refer to a sequence in which two or more identical or extremely similar nucleotide sequences are repeated. A repeated sequence includes but is not limited to a tandem repeat or interspersed repeat. The repeated sequence may be a direct repeat or inverted repeat. Tandem repeats are repeated sequences which are directly adjacent to each other. Interspersed repeats are repeated sequences which are found in different locations. Direct repeats occur when a nucleotide sequence is repeated with the same directionality. Inverted repeats occur when a nucleotide sequence is repeated in the inverse direction. When there are no nucleotides separating the inverted repeat, the sequence is called a palindromic repeat. The repeated sequence may be at least 2, 5, 10, 25, 50, 75, 100, 250, 500 or 750 nucleotides long. The repeated sequence may be 2-1000 nucleotides long, 2-500 nucleotides long, 2-250 nucleotides long, 2-100 nucleotides long, 5-1000 nucleotides long, 5-500 nucleotides long, 5-250 nucleotides long, 5-100 nucleotides long. Preferably, the repeated sequence is 2-1000 nucleotides long.

The at least one inhibitory region may comprise a homopolymeric sequence. Homopolymeric sequence may refer to a sequence in which there are at least 4 repeats of the same base. Preferably, a homopolymeric sequence comprises at least 8 repeats of the same base.

If the at least one inhibitory region comprises a homopolymeric region consisting of A, T, C or G, the ssNA molecule(s) may comprise a complementary homopolymeric region consisting of T, A, G or C respectively. The at least one inhibitory region may comprise a polyA sequence and thus the ssNA molecule(s) may comprise a polyT sequence. The at least one inhibitory region may comprise a polyT sequence and thus the ssNA molecule(s) may comprise a polyA sequence. The at least one inhibitory region may comprise a polyG sequence and thus the ssNA molecule(s) may comprise a polyC sequence. The at least one inhibitory region may comprise a polyC sequence and thus the ssNA molecule(s) may comprise a polyG sequence.

The at least one inhibitory region may comprise a sequence that is prone to forming an inhibitory structure. The inhibitory structure may comprise (i) a structure that inhibits strand displacement by a polymerase; and/or (ii) a structure that inhibits a primer annealing to the DNA template molecule. The inhibitory structure may have secondary, tertiary, and/or quaternary structure. The inhibitory structure may be a non B-DNA structure. The inhibitory structure may comprise A-DNA, Z-DNA, H-DNA, a G-quadruplex, and/or a DNA triplex. The inhibitory structure(s) may be a DNA loop, cruciform, hairpin structure, and/or DNA tetraplex.

Inhibitory regions may comprise a CAG promoter sequence; an insulator; a homopolymeric sequence and/or an inverted terminal repeat. Insulator may refer to a type of cis-regulatory element typically 300 bp to 2000 bp in length. An insulator may function either as an enhancer-blocker or a barrier, or both.

The at least one inhibitory region may have a length of at least 4 bp, 5 bp, 6 bp, 7 bp, 8 bp. Preferably, the at least one inhibitory region has a length of at least 8 bp.

The inhibitory region may comprise: a CAG promoter sequence; an insulator; a homopolymeric sequence and/or an inverted terminal repeat.

The first and second adaptor molecules may be identical molecules or they may be different molecules. For example, the first adaptor molecule and/or the second adaptor molecule may comprise a hairpin. The first adaptor molecule and/or the second adaptor molecule may be double-stranded linear nucleic acid molecules comprising one or more nuclease-resistant nucleotides. The first adaptor molecule may comprise a hairpin and the second adaptor molecule may be a double-stranded linear nucleic acid molecule comprising one or more nuclease-resistant nucleotides. Thus, the adaptor-ligated DNA product may be resistant to nuclease (e.g. exonuclease) digestion.

The first adaptor molecule may be hybridized to the first end of the linear double-stranded region. The second adaptor molecule may be hybridized to the second end of the linear double-stranded region. The first adaptor molecule may be ligated to the first end of the linear double-stranded region. The second adaptor molecule may be ligated to the second end of the linear double-stranded region. The first adaptor molecule may be hybridized and ligated to the first end of the linear double-stranded region. The second adaptor molecule may be hybridized and ligated to the second end of the linear double-stranded region. The first adaptor molecule may be appended to the first end of the linear double-stranded region via a linker or spacer molecule. The second adaptor molecule may be appended to the second end of the linear double-stranded region via a linker or spacer molecule.

The linker or spacer may be at least 10, at least 20, at least 30, at least 40, at least 50, at least 60, at least 70, at least 80, at least 90, at least 100, at least 125, at least 150, at least 175, or at least 200 base pairs long.

The invention also provides an adaptor-ligated DNA product comprising a linear double-stranded region comprising an inhibitory region, wherein the inhibitory region inhibits complete and/or faithful amplification of the linear double-stranded region, wherein the linear double-stranded region is ligated at a first end to a first adaptor molecule and ligated at a second end to a second adaptor molecule, wherein the first and second adaptor molecules confer exonuclease resistance to the adaptor-ligated DNA product. The inhibitory region may be any inhibitory region described herein including but not limited to regions having:
i) a GC content of at least 60%;
ii) a repeated sequence;
ii) a homopolymeric sequence (such as a polyG sequence); and/or
iv) a sequence that is prone to forming an inhibitory structure (such as (i) a structure that inhibits strand displacement by a polymerase; and/or (ii) a structure that inhibits a primer annealing to the DNA template molecule).

The invention also provides an adaptor-ligated DNA product comprising a linear double-stranded region comprising an inhibitory region, wherein the inhibitory region inhibits complete and/or faithful amplification of the linear double-stranded region, wherein the linear double-stranded region is ligated at a first end to a first adaptor molecule and ligated at a second end to a second adaptor molecule, wherein the first and second adaptor molecules confer exonuclease resistance to the adaptor-ligated DNA product and wherein the inhibitory region comprises: a polyG sequence and/or an insulator.

The invention also provides an adaptor-ligated DNA product comprising a linear double-stranded region comprising an inhibitory region, wherein the inhibitory region inhibits complete and/or faithful amplification of the linear double-stranded region, wherein the linear double-stranded region is ligated at a first end to a first adaptor molecule and ligated at a second end to a second adaptor molecule, wherein the first and second adaptor molecules confer exonuclease resistance to the adaptor-ligated DNA product and wherein the inhibitory region comprises: a CAG promoter sequence; an insulator; a homopolymeric sequence and/or an inverted terminal repeat.

In the adaptor-ligated DNA product:
a) the linear double-stranded region may be closed at a first end by the first adaptor molecule and closed at a second end by the second adaptor molecule;
b) the first and second adaptor molecules may be nucleic acid molecules that comprise one or more nuclease-resistant nucleotides; or
c) the first adaptor molecule may be a nucleic acid molecule that comprises one or more nuclease-resistant nucleotides, and wherein the linear double-stranded region may be closed at the second end by the second adaptor molecule.

The first adaptor molecule and/or the second adaptor molecule may comprise one or more locked nucleic acids (LNAs).

The first adaptor molecule and/or the second adaptor molecule may comprise one or more protected nucleotides (i.e. nuclease-resistant nucleotides), such as phosphorothioated nucleotides.

The first and second adaptor molecules may comprise one or more phosphorothioated nucleotides, such that, once the adaptor molecules are appended (e.g. ligated) to the digested DNA product, the adaptor-ligated DNA product is resistant to nuclease digestion or has improved or enhanced resistance to nuclease digestion. The adaptor-ligated DNA product may be resistant to 3'-end exonuclease digestion (e.g. by exonuclease III) and/or 5'-end exonuclease digestion (e.g. by exonuclease VIII).

The adaptor molecule may comprise a plurality of phosphorothioated nucleotides. For example, the adaptor molecules may comprise at least 2, at least 3, at least 4, at least 5, at least 6, at least 7, at least 8, at least 9, at least 10, at least 11, at least 12, at least 13, at least 14, at least 15 or at least 16 phosphorothioated nucleotides in each strand.

The adaptor molecule may be a nucleic acid adaptor molecule. The adaptor molecule may be double-stranded. The adaptor molecule may comprise a portion that is double-stranded.

The first and/or second adaptor molecules may comprise at least 3, at least 4, at least 5, at least 6, at least 7, at least 8, at least 9, at least 10, at least 11, at least 12, at least 13, at least 14, at least 15, or at least 16 base pairs.

The adaptor molecule may comprise a plurality of phosphorothioated nucleotides in each strand. For example, the adaptor molecules may comprise at least 2, at least 3, at least 4, at least 5, at least 6, at least 7, at least 8, at least 9, at least 10, at least 11, at least 12, at least 13, at least 14, at least 15 or at least 16 phosphorothioated nucleotides in each strand.

The adaptor molecule may comprise a plurality of phosphorothioated nucleotides at internal positions in each strand. For example, the adaptor molecules may comprise at least 1, at least 2, at least 3, at least 4, at least 5, at least 6, at least 7, at least 8, at least 9, at least 10, at least 11, at least 12, at least 13, at least 14, at least 15 or at least 16 phosphorothioated nucleotides at internal positions in each strand. Preferably, the adaptor molecule comprises at least 2 phosphorothioated nucleotides at internal positions in each strand.

The internal positions may not be located between the second and penultimate nucleotide of the adaptor molecule. The internal positions may be any position in the adaptor molecules other than the last nucleotide at the end of each strand.

The adaptor molecule may comprise at least 5%, at least 10%, at least 20%, at least 30%, at least 40%, at least 50%, at least 60%, at least 70%, at least 80%, at least 90%, at least 95%, or at least 100% of protected nucleotides.

The nucleotides resistant to exonuclease digestion (i.e. protected nucleotides) may be phosphorothioated nucleotides of at least one type. For example, the at least one type of phosphorothioated nucleotides is α-S-dATP (i.e. 2'-deoxyadenosine-5'-(a-thio)-triphosphate), α-S-dCTP (i.e. 2'-deoxycytidine-5'-(α-thio)-triphosphate), α-S-dGTP (i.e. 2'-deoxyguanosine-5'-(α-thio)-triphosphate), α-S-dTTP (i.e. 2'-deoxythymidine-5'-(α-thio)-triphosphate), α-S-dUTP (i.e. 2'-deoxyuridine-5'-(α-thio)-triphosphate), and/or uridine 2', 3'-cyclophosphorothioate.

The adaptor molecules may comprise at least two types of phosphorothioated nucleotides. For example, the at least two types of phosphorothioated nucleotides are: α-S-dATP and α-S-dCTP, α-S-dATP and α-S-dGTP, α-S-dATP and α-S-dTTP, α-S-dCTP and α-S-dGTP, α-S-dCTP and α-S-dTTP, or α-S-dGTP and α-S-dTTP.

The adaptor molecules may comprise at least three types of phosphorothioated nucleotides. For example, the at least three types of phosphorothioated nucleotides are:
b. α-S-dATP, α-S-dCTP and α-S-dGTP;
c. α-S-dATP, α-S-dCTP and α-S-dTTP;
d. α-S-dATP, α-S-dGTP and α-S-dTTP; or
e. α-S-dCTP, α-S-dGTP and α-S-dTTP.

The adaptor molecules may comprise at least four types of phosphorothioated nucleotides. For example, the at least four types of protected nucleotides are α-S-dATP, α-S-dCTP, α-S-dGTP and α-S-dTTP.

The phosphorothioated nucleotides may be Sp-isomers, Rp-isomers or a mixture of both Sp- and Rp-isomers.

The nucleotides resistant to exonuclease digestion (i.e. protected nucleotides) may be MOE nucleotides of at least one type, or at least two, three or four types. For example, the MOE nucleotides may be 2'-O-methoxy-ethyl guanosine, 2'-O-methoxy-ethyl cytidine, 2'-O-methoxy-ethyl adenosine, and/or 2'-O-methoxy-ethyl thymidine.

The first end of the digested DNA product may be complementary to a portion of the first adaptor molecule. The second end of the digested DNA product may be complementary to a portion of the second adaptor molecule. The first end and/or the second end of the digested DNA product may be generated by endonuclease digestion as described herein.

As used herein, the term "complementary" refers to the pairing of nucleotide sequences according to Watson/Crick pairing rules. For example, a sequence 5'-GCGGTCCCA-3' has the complementary sequence of 5'-TGGGACCGC-3'. A complement sequence can also be a sequence of RNA complementary to the DNA sequence.

The first adaptor molecule and/or the second adaptor molecule may comprise a functional portion. The functional portion may be a binding molecule, a targeting sequence, or a probe.

The functional portion may be a probe. As used herein, the term "probe" refers to a fragment of DNA, RNA or DNA/RNA chimera of variable length (e.g. 3-1000 bases long), which is used to detect the presence of target nucleotide sequences that are complementary to the sequence in the probe.

Typically, the probe hybridizes to single-stranded nucleic acid whose base sequence allows probe-target base pairing due to complementarity between the probe and target. Thus, the functional portion may be a DNA sequence, a RNA sequence or a DNA/RNA chimera sequence. As used herein, the term "complementary" refers to the pairing of nucleotide sequences according to Watson/Crick pairing rules. For example, a sequence 5'-GCGGTCCCA-3' has the complementary sequence of 5'-TGGGACCGC-3'. A complement sequence can also be a sequence of RNA complementary to the DNA sequence.

The functional portion may be a binding molecule. The term "binding molecule" refers to any molecule capable of binding to the adaptor-ligated DNA product described herein and/or that is capable of binding to a further molecule or target. The binding molecule may be a protein, a polypeptide, or a peptide. The binding molecule may be an antibody, such as a monoclonal antibody or a polyclonal antibody. The binding molecule may be an antibody fragment.

The functional portion may facilitate detection of the DNA product by binding to capture molecules (e.g. capture antibodies bound by protein-protein interactions). The functional portion may bind to a cell target, for example, a cell receptor.

The functional portion may be a label. The 'label' can be any chemical entity which enable the detection of the double-stranded nucleic acid molecule via, physical, chemical and/or biological means. The label may be a chromophore, a fluorophore and/or a radioactive molecule.

The functional portion may be a targeting sequence. The targeting sequence may be a fragment of DNA or RNA of variable length, which is used to target the DNA product to a specific location in a cell. The targeting sequence may be used to increased transfection efficiency of non-viral gene delivery by virtue of enhanced nuclear import of the adaptor-ligated DNA product. For example, the targeting sequence may be a DNA nuclear targeting sequences (i.e. a recognition sequence for endogenous DNA-binding proteins), such as SV40 enhancer sequence (preferably downstream from the cassette).

To facilitate detection and/or quantification of the adaptor-ligated DNA product, the functional portion may comprise a fluorophore, a radioactive compound or a barcode.

The functional portion may also facilitate DNA sequencing. For example, the functional portion may be a sequencing adapter. The term "sequencing adapter" is intended to encompass one or more nucleic acid domains that include at least a portion of a nucleic acid sequence (or complement thereof) utilized by a sequencing platform of interest, such as a sequencing platform provided by Illumina^{®} (e.g. the HiSeq^{™}, MiSeq^{™} and/or Genome Analyzer^{™} sequencing systems), Oxford Nanopore^{™} Technologies (e.g. the MinION sequencing system), Ion Torrent^{™} (e.g. the Ion PGM^{™} and/or Ion Proton^{™} sequencing systems), Pacific Biosciences (e.g. the PACBIO RS II sequencing system); Life Technologies^{™} (e.g. a SOLiD sequencing system), Roche (e.g. the 454 GS FLX+ and/or GS Junior sequencing systems), or any other sequencing platform of interest.

The first adaptor molecule and/or the second adaptor molecule may comprise an inverted terminal repeat sequence. The inverted terminal repeat sequences of the first adaptor molecule and the second adaptor molecule may be symmetrical (i.e. have the same symmetrical three-dimensional organization with respect to each other) or asymmetrical (i.e. have different three-dimensional organization with respect to each other). The inverted terminal repeat sequences of the first adaptor molecule and the second adaptor molecule may be from the same or different serotypes. An inverted terminal repeat sequence may comprise a terminal resolution site and a Rep binding site.

The first adaptor molecule and/or the second adaptor molecule may comprise an aptamer.

The first adaptor molecule and/or the second adaptor molecule may confer resistance to the nuclease digestion, such as exonuclease digestion (e.g. exonuclease I and/or exonuclease III digestion).

The first adaptor molecule may be a nucleic acid adaptor molecule. The second adaptor molecule may be a nucleic acid adaptor molecule. The first adaptor molecule and/or the second adaptor molecule may comprise a self-complementary element which creates a loop, such as a hairpin loop or a stem loop. Thus, the first adaptor molecule may comprise a hairpin or a stem-loop. The second adaptor molecule may comprise a hairpin or a stem-loop. Both the first and second adaptor molecules may comprise a hairpin or a stem-loop. The adaptor molecules may each comprise a double-stranded portion comprising a sense strand and an antisense strand, wherein the sense strand and the antisense strand are linked together in a hairpin such that the sense strand is hybridized to the antisense strand. The double-stranded portion of an adaptor may comprise a 3' overhang or a 5' overhang of at least 1, at least 2, at least 3, at least 4, or at least 5 nucleotides. Preferably the 3' overhang or the 5' overhang is 4-8 nucleotides. Each end of the linear double-stranded region (or linear portion of the double-stranded DNA molecule) may comprise a 3' or a 5' overhang. A portion of the first adaptor molecule (e.g. the overhang) may be complementary to the first end of the linear double-stranded region. A portion of the second adaptor molecule may be complementary to the second end of the linear double-stranded region.

The adaptor-ligated DNA product may be a closed linear DNA product, optionally a covalently closed linear DNA product. Thus, in embodiments where the adaptor molecules comprise a loop (e.g. a hairpin), the adaptor molecules close the ends of the linear double-stranded region forming a covalently closed linear DNA product. When only one of the first or second adaptor molecules comprises a loop, a partially closed linear DNA molecule will result.

The adaptor-ligated DNA product may be a linear double-stranded DNA molecule, a closed linear DNA molecule or a partially closed linear DNA molecule. A linear double-stranded DNA molecule may result from the first and second adaptor molecules being linear double-stranded DNA molecules. A closed linear DNA molecule may result from the first and second adaptor molecules comprising a hairpin or stem-loop. A partially closed linear DNA molecule may result from one of the first or second adaptor molecules being a linear double-stranded DNA molecule and other comprising a hairpin or stem-loop. Linear double-stranded adaptors may comprise protected nucleotides as described herein thereby conferring exonuclease resistance on the adaptor-ligated DNA product. Methods for producing closed linear DNA products, linear double-stranded DNA products and partially closed linear DNA products are provided in WO2023/006978A1, which is incorporated herein by reference.

The DNA products of the invention (e.g. the adaptor-ligated DNA products) can be used for expression in a host cell, for example, in DNA vaccines. DNA vaccines typically encode a modified form of an infectious organism's DNA. DNA vaccines are administered to a subject where they then express the selected protein of the infectious organism, initiating an immune response against that protein which is typically protective. DNA vaccines may also encode a tumor antigen in a cancer immunotherapy approach.

The DNA products of the invention (e.g. the adaptor-ligated DNA products) may be other types of therapeutic DNA molecules e.g. those used in gene therapy. For example, such DNA molecules can be used to express a functional gene where a subject has a genetic disorder caused by a dysfunctional version of that gene. Examples of such diseases include sickle cell anemia, cystic fibrosis, Huntington disease, Duchenne's Muscular Dystrophy, Hemophilia A, α1-antitrypsin deficiency, primary ciliary dyskinesia, or respiratory distress syndrome of prematurity. Other diseases where gene therapy may be useful include metabolic diseases, respiratory diseases, inflammatory diseases, autoimmune, chronic and infectious diseases, including such disorders as AIDS, cancer, neurological diseases, cardiovascular disease, hypercholesterolemia, various blood disorders including various anemia, thalassemia and hemophilia, and emphysema. For the treatment of solid tumors, genes encoding toxic peptides (i.e., chemotherapeutic agents such as ricin, diphtheria toxin and cobra venom factor), tumor suppressor genes such as p53, genes coding for mRNA sequences which are antisense to transforming oncogenes, antineoplastic peptides such as tumor necrosis factor (TNF) and other cytokines, or transdominant negative mutants of transforming oncogenes, may be expressed.

The DNA products of the invention (e.g. the adaptor-ligated DNA products) can be used for the production of viral vectors, such as AAV vectors. The DNA products of the invention (e.g. the adaptor-ligated DNA products) can be used as a DNA template for the production of mRNA via *in vitro* transcription.

Each aspect or embodiment as defined herein may be combined with any other aspect(s) or embodiment(s) unless clearly indicated to the contrary. In particular, any feature indicated as being preferred or advantageous may be combined with any other feature or features indicated as being preferred or advantageous.

The foregoing detailed description has been provided by way of explanation and illustration, and is not intended to limit the scope of the appended claims. Many variations in the presently preferred embodiments illustrated herein will be apparent to one of ordinary skill in the art, and remain within the scope of the appended claims and their equivalents.

### BRIEF DESCRIPTION OF THE DRAWINGS

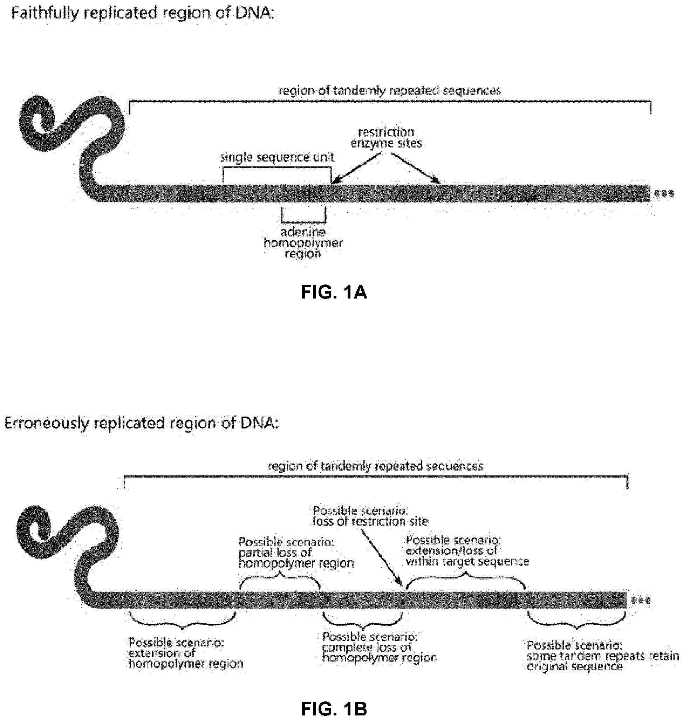

**FIG. 1A** depicts the scenario where a DNA template is faithfully amplified generating long strands of DNA made up of series of tandemly repeating target sequence with no alterations from the template sequence. Each repeat contains a restriction site that will allow digestion of the tandem repeat region into identical monomer sequence units.
**FIG. 1B** depicts the scenario where a DNA template is not faithfully amplified due to the presence of inhibitory regions. This leads to truncation, extension or complete loss of homopolymeric regions; truncation, extension or partial loss of target regions; mis-replication or loss of restriction site regions. Erroneous amplification can result in a population of monomer products all containing identical replication errors, it can also result in a mixed population of monomer products containing correct and incorrect sequences, it can also result in a mixed population of monomer products all containing a range of different incorrect sequences, it can also result in synthesis of tandemly repeating product that cannot be digested into its monomer units due to incorrectly formed/replicated restriction site regions. The presence of inhibitory regions can also result in faithful amplification with below expected yields or with no amplification taking place, resulting in no DNA product.

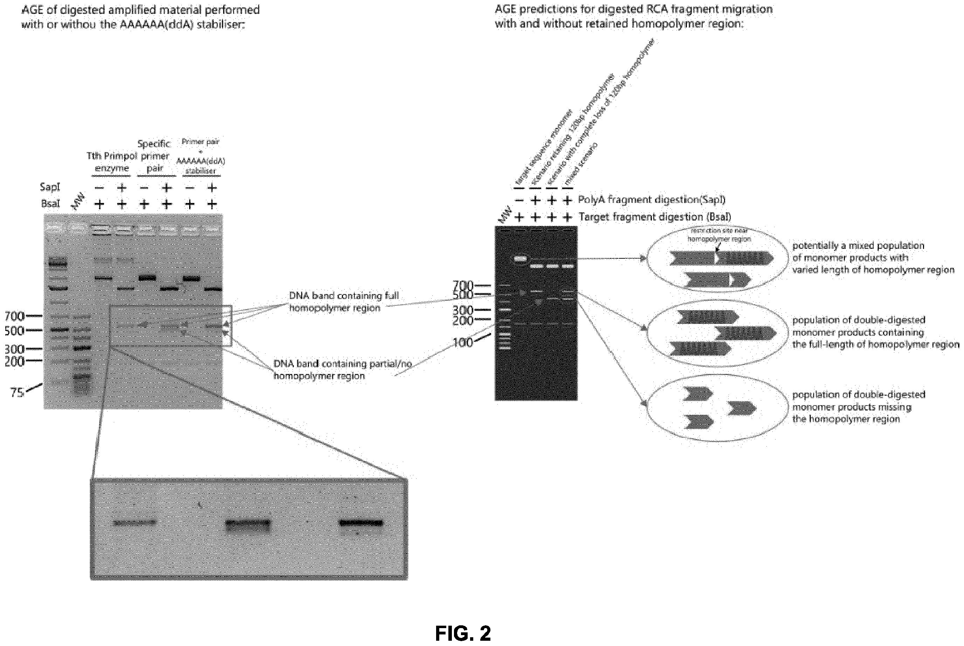

**FIG. 2** depicts the agarose gel electrophoresis analysis of DNA amplified by RCA using *Tth*PrimPol or a specific primer pair followed by digestion with Sapl and Bsal. The DNA template comprises a 120nt A:T homopolymer region which is not faithfully amplified when using the specific primer pair. A short polyA oligonucleotide recovers faithful amplification of the A:T homopolymer region. Also shown in an *in silico* prediction generated using SnapGene software AGE simulation.

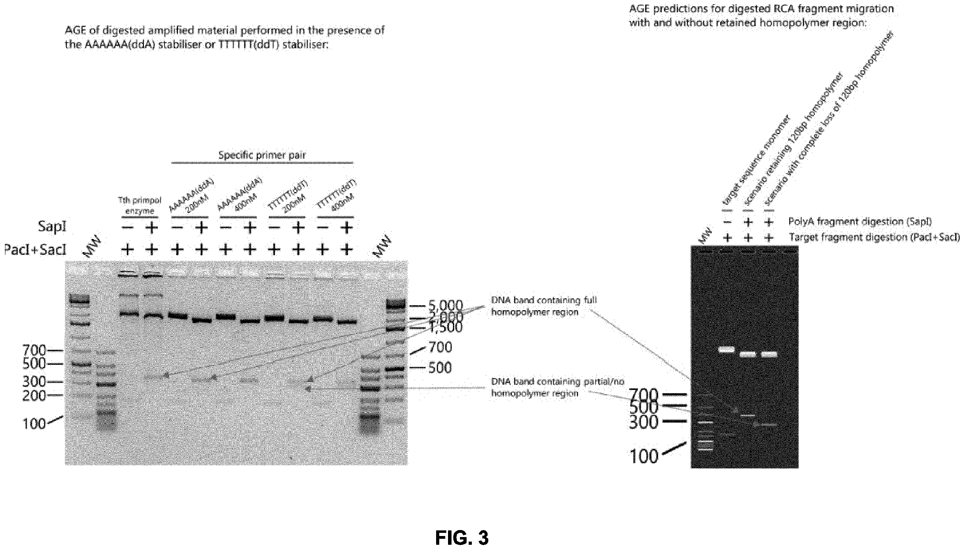

**FIG. 3** depicts the agarose gel electrophoresis analysis of DNA amplified by RCA using *Tth*PrimPol or a specific primer pair followed by digestion with Pacl, Sacl and Sapl. The DNA template comprises a 120nt A:T homopolymer region which is not faithfully amplified when using the specific primer pair. A short polyA oligonucleotide, but not a polyT oligonucleotide, recovers faithful amplification of the A:T homopolymer region. Also shown in an *in silico* prediction generated using SnapGene software AGE simulation.

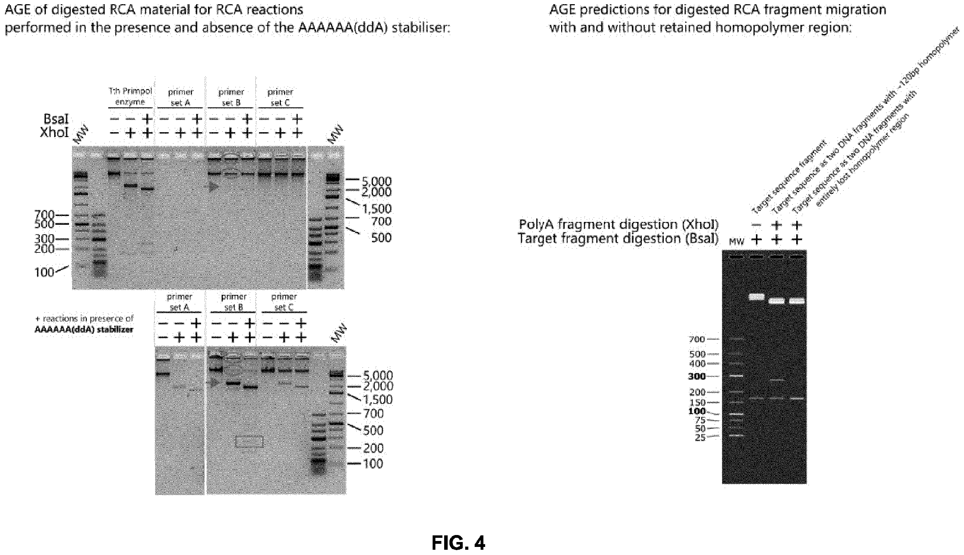

**FIG. 4** depicts the agarose gel electrophoresis analysis of DNA amplified by RCA using *Tth*PrimPol or a specific primer pair (primer set A, B or C) followed by digestion with Bsal and Xhol. The DNA template comprises a 120nt A:T homopolymer region which is not faithfully amplified using the specific primer pairs (see upper panel). A short polyA oligonucleotide recovers faithful amplification of the A:T homopolymer region. Also shown in an *in silico* prediction generated using SnapGene software AGE simulation.

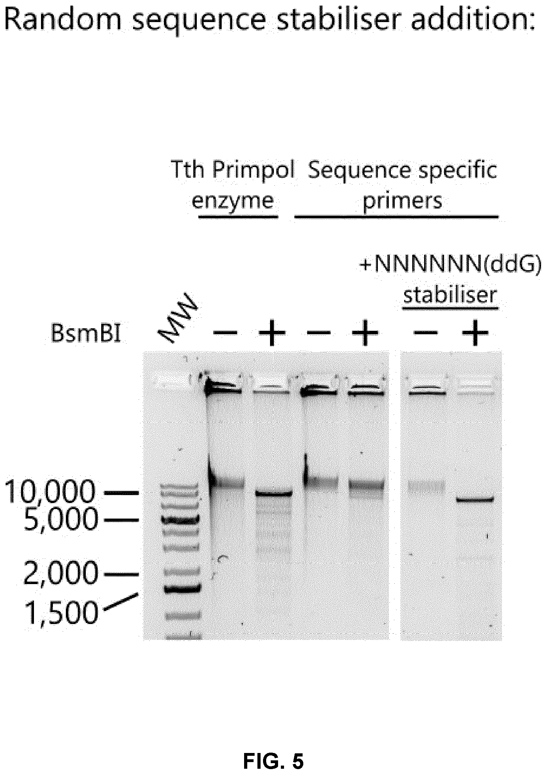

**FIG. 5** depicts the agarose gel electrophoresis analysis of DNA amplified by RCA using *Tth*PrimPol or a specific primer pair followed by digestion with BsmBl. The DNA template is not faithfully amplified using the specific primer pair. Random heptamers recover faithful amplification of the DNA template.

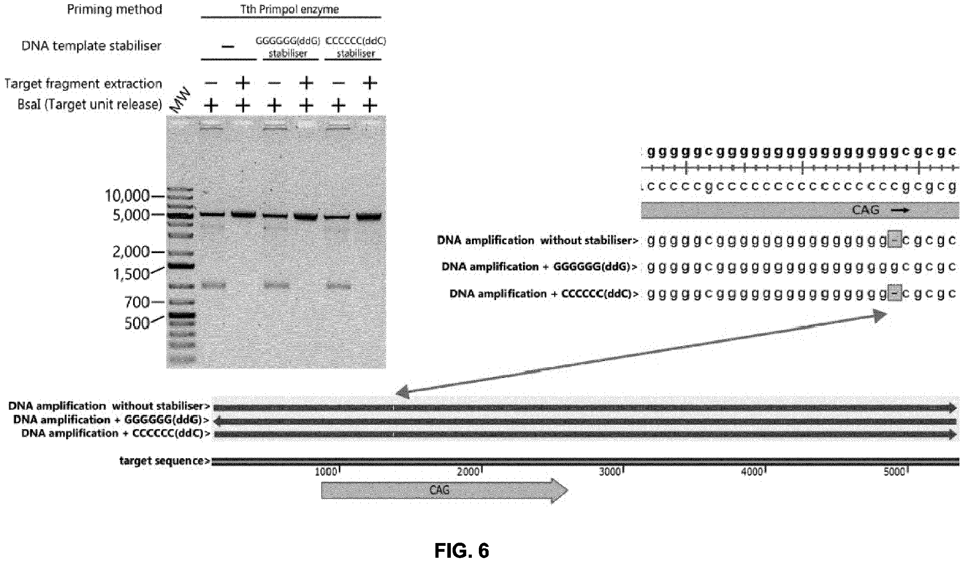

**FIG. 6** depicts the agarose gel electrophoresis analysis of DNA amplified by RCA using *Tth*PrimPol followed by digestion with Bsal. The DNA template comprises a G:C homopolymer region which is not faithfully amplified. A short polyG oligonucleotide, but not a polyC oligonucleotide, recovers faithful amplification of the G:C homopolymer region as shown by the sequencing data.

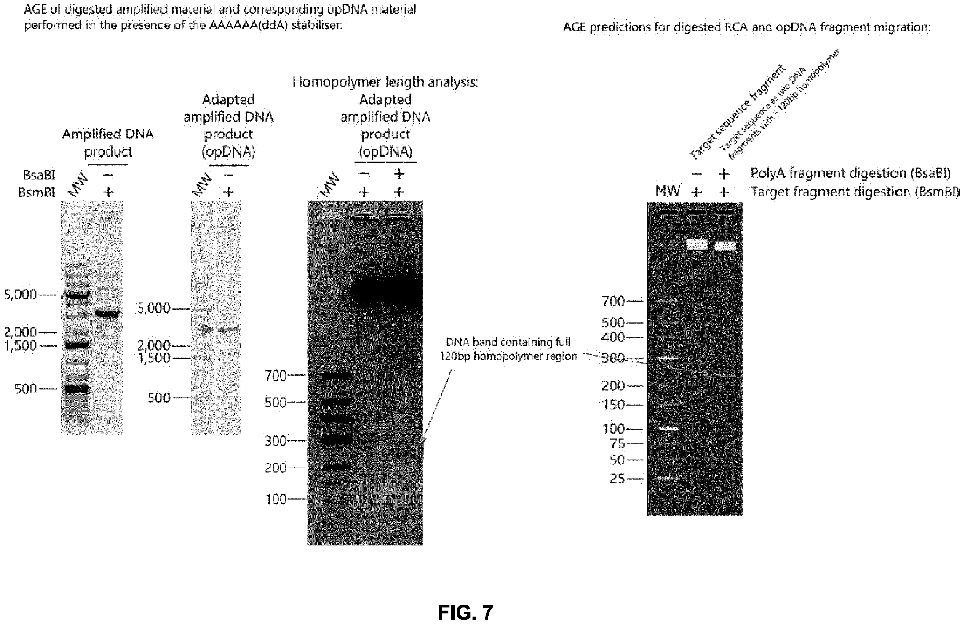

**FIG. 7** depicts the agarose gel electrophoresis analysis of DNA amplified by RCA using a specific primer pair in the presence of a polyA stabiliser followed by adaptor ligation.

### EXAMPLES

### Example 1: PolyA stabilisers in the amplification of A:T homopolymer regions

### Method:

A DNA construct sized ~2000bp containing a 120bp poly-Adenine tail region was amplified using rolling circle amplification (RCA) with QualiPhi^{®} (a chimeric form of Phi29 DNA polymerase, 4basebio). Priming of the template DNA strands was either performed using *Tth*PrimPol enzyme, that primes DNA in sequence-independent fashion, or with a pair of sequence-specific primers. The reaction was incubated under the following RCA conditions:
Before amplification, circularized DNA template was first denatured by adding 1 volume of buffer D (400 mM KOH, 10 mM EDTA) and incubated for 3 min at room temperature. Samples were then neutralized by adding 1 volume of buffer N (400 mM HCl, 600 mM Tris-HCl pH 7.5). Rolling circle amplification was performed in 1 ml reaction volume, 100 µl TruePrime WGA reaction buffer 10x (4basebio), 50 µl denatured DNA sample, 4.6µl TthPrimPol (21.8 µM), 16 µl QualiPhi Phi29 DNA polymerase (12,5 µM), 2.5 µl Pyrophosphatase (2.47 µM) and 40 µl dNTPs (25 mM). Incubation time and temperature: 20 hours at 30°C, followed by 10 min at 65°C.

Additionally, each primer-mediated RCA reaction was supplemented with 200nM of Adenine Heptamer containing 3' dideoxy, chain terminating nucleotide AAAAAA(ddA). The stabilizer molecule, regardless of the sequence can be added either during the template denaturation step or alongside of the DNA priming agent whether this would be a random short primer collection, sequence-specific primer set, or DNA primase enzyme._

The amplified RCA material was digested by Bsal restriction enzyme to release target sequence and Sapl restriction enzyme to generate a smaller fragment containing mainly the A:T homopolymer sequence. This double digestion provides better resolution between bands of similar size in agarose gel electrophoresis (AGE).

### Results:

Amplified RCA material, containing long continuous repeats of the target sequence, was digested with restriction enzyme that releases target sequence in the form of monomers. As shown in the AGE in FIG. 2, TthPrimPol mediated RCA can generate a major target fragment band at 2kb upon Bsal digestion. In the case of sequence specific primers, a target product band is also generated successfully.

The size assessment of the A:T-homopolymer region was performed by further digestion of the target sequence, with Sapl restriction enzyme to generate a smaller fragment containing the A:T-homopolymer region, so that the variability of the homopolymer region length could be more readily observed. Due to the length of the homopolymer region, DNA sequencing cannot accurately identify the length of the A:T homopolymer tail in this example. If the whole homopolymer region is retained, the Sapl fragment size would be expected at ~550bp as shown in the Snapgene simulation. *Tth*PrimPol mediated RCA generated a Sapl fragment appearing as a single band at ~550bp, indicating the presence of a uniform, complete 120bp homopolymer region within each RCA product monomer unit.

In the case of primer-mediated RCA, Sapl digestion of the RCA monomer products results in two DNA bands at ~550bp and ~500bp (as highlighted with two arrows in **FIG. 2****),** indicating that at least two populations of products are formed, a subset that fully retained the 120bp A:T homopolymer region and a subset where a portion of the homopolymer region is lost, resulting in multiple populations of RCA products.

In primer-mediated RCAs supplemented by AAAAAA(ddA) stabiliser, upon Sapl digestion of the RCA monomer products two bands are generated at ~550bp and ~500bp. However, the products are heavily skewed in favor of the ~550bp product (shown in the zoomed-in panel). Thus, the AAAAAA(ddA) stabiliser improves faithful amplification and retention of the A:T-homopolymer region.

### Example 2: PolyA and PolyT stabilisers in the amplification of A:T homopolymer regions

### Method:

A DNA construct sized ~2000bp containing a 120bp poly-Adenine tail region was amplified using rolling circle amplification (RCA) with QualiPhi^{®} (a chimeric form of Phi29 DNA polymerase, 4basebio). Priming of the template DNA strands was either performed using *Tth*PrimPol enzyme, that primes DNA in sequence-independent fashion, or with a pair of sequence-specific primers. The reaction was incubated at 30°C for 20 hours under the same RCA conditions as provided above for Example 1.

The DNA template utilized in the RCA consists of two complementary, denatured DNA strands, where each strand can exhibit a different propensity for inhibiting amplification, e.g. the first strand may not inhibit amplification while the second strand may inhibit amplification. Therefore, in this example each primer-mediated RCA was supplemented with 200nM or 400nM of either Adenine Heptamer containing 3' dideoxy, chain terminating nucleotide (AAAAAA(ddA)) or Thymine Heptamer containing 3' dideoxy, chain terminating nucleotide (TTTTTT(ddT)) to identify which strand may require the presence of complementary stabiliser molecules.

The amplified RCA material was digested with a combination of Sacl and Pacl restriction enzymes to release the target monomeric sequence, and Sapl restriction enzyme to further isolate the A:T homopolymer sequence.

### Results:

Amplified RCA material, containing long continuous repeats of the target sequence, was digested with restriction enzyme to release target sequence in the form of monomers. As shown in the AGE in **FIG. 3****,** *Tth*PrimPol mediated RCA generates a major target fragment band at 2kb upon Sacl and Pacl digestion. In the case of sequence-specific primer-mediated RCA reactions supplemented with stabiliser molecules; the target product band is also generated successfully.

The size assessment of A:T-homopolymer region was performed by further digestion of the target monomer products, with Sapl restriction enzyme, to generate a smaller fragment containing A:T-homopolymer region. If the whole homopolymer region is retained, the fragment size would be expected at ~390bp as illustrated in the AGE simulation. As shown in **FIG. 3****,** *Tth*PrimPol mediated RCA generates a single uniform band at ~390bp upon Sapl digestion, indicating the presence of the complete 120bp A:T homopolymer region within each copy of the RCA monomer product.

Comparing primer-mediated RCAs supplemented either with AAAAAA(ddA) stabiliser or TTTTTT(ddT) stabiliser, the polyA (AAAAAA(ddA)) stabiliser at both 200nM and 400nM generates a uniform population of RCA products with each copy fully retaining the 120bp A:T homopolymer region and forming a single expected band at ~390bp after Sapl digestion. Meanwhile, supplementation with polyT (TTTTTT(ddT)) stabiliser results in a mixed population of RCA monomer products of which a subset of the amplified DNA has lost a portion of the A:T homopolymer region (as indicated by DNA bands both at and below ~390bp upon Sapl digestion). This result demonstrates that in this instance the DNA template strand containing the poly-Thymine homopolymer sequence requires stabilization with complementary polyA stabilizer to ensure faithful amplification.

### Example 3: PolyA stabilisers in the amplification of A:T homopolymer regions

### Method:

A DNA construct sized ~2100bp containing a 120bp poly-Adenine tail region was amplified using rolling circle amplification (RCA) with QualiPhi^{®} (a chimeric form of Phi29 DNA polymerase, 4basebio). Priming of the template DNA strands was either performed via *Tth*PrimPol enzyme, that primes DNA in sequence-independent fashion, or with a pair of sequence-specific primers (primer set A, B or C). Reaction was incubated at 30°C for 20 hours under the same RCA conditions as provided for Example 1.

Additionally, each primer-mediated RCA was supplemented with 200nM of Adenine Heptamer containing 3' dideoxy, chain terminating nucleotide AAAAAA(ddA).

Amplified RCA material was digested by Bsal restriction enzyme to release target sequence and Xhol restriction enzyme to generate a smaller DNA fragment containing the A:T homopolymer sequence.

### Results:

Amplified RCA material, containing long continuous repeats of the target sequence, was digested with Bsal restriction enzyme to release target sequence in the form of monomers. As shown in the upper AGE in **FIG. 4****,** only *Tth*PrimPol mediated RCA generated a dominant target fragment band at 2.1kb upon Bsal digestion. In the case of primer mediated RCA, primer set A led to no amplification taking place. Primer set B and primer set C led to insufficient or no correctly formed target material that could be digested into monomer forms. The RCA reaction mediated by primer set B, generated two populations of RCA products. Faithfully amplified target DNA that can be digested into monomer units is indicated with an arrow in the upper AGE in **FIG. 4****.** Indigestible high molecular weight DNA strands of imperfect tandem repeats caused by erroneous amplification are circled in the upper AGE in **FIG. 4****.**

The size assessment of the 120bp A:T-homopolymer region was performed by further digestion of the target sequence, with Xhol restriction enzyme, to generate a smaller A:T-homopolymer-containing region of expected size ~270bp. As shown in the upper AGE in **FIG. 4****,** *Tth*PrimPol mediated RCA generates a band at ~270bp, indicating the presence of the 120bp homopolymer region. Homopolymer presence cannot be assessed for the primer-mediated RCAs as the amount of RCA-generated target material is too low to be able to produce detectable <270bp bands on AGE after Xhol digestion.

In the primer-mediated RCAs supplemented with AAAAAA(ddA) stabiliser, an improvement is observed in all three primer-mediated cases, with each RCA reaction now capable of amplification and generating the target DNA band of 2,1kb. The most noticeable effect is exemplified in RCA utilizing primer set B which generates a dominant target band upon Bsal digestion of RCA material (indicated with an arrow in the lower AGE of **FIG. 4****).** In the primer set B-mediated RCA reaction supplemented with polyA (AAAAAA(ddA)) stabilizer molecules, the proportion of the target material in relationship to the indigestible RCA material is now heavily skewed in favor of the target material unlike the RCA reaction utilizing primer pair B alone. The stabilizer-containing reaction is also able to retain the full 120bp homopolymer region as the expected ~270bp DNA band forms upon further digestion with Xhol (highlighted in the rectangle in the lower AGE of **FIG. 4****).**

### Example 4: Random stabilisers in the amplification of DNA

### Method:

A DNA construct sized ~9300bp containing no homopolymer regions was amplified using rolling circle amplification (RCA) with QualiPhi^{®} (a chimeric form of Phi29 DNA polymerase, 4basebio). Priming of the template DNA strands was either performed using *Tth*PrimPol enzyme, that primes DNA in sequence-independent fashion, or with a pair of sequence-specific primers. Reaction was incubated at 30°C for 20 hours under the same RCA conditions as provided for Example 1.

Additionally, primer-mediated RCA was supplemented with 600nM random sequence stabiliser containing a mixture of oligonucleotides made up of 6 random nucleotides followed by Guanosine nucleotide containing 3' dideoxy chain terminating modification (NNNNNN(ddG)).

The amplified RCA material was digested by BsmBl restriction enzyme to release the target sequence in a form of monomers.

### Results:

Amplified RCA material, containing long continuous tandem repeats of the target sequence, was digested with restriction enzyme to release target sequence in the form of monomers. As shown in the AGE of **FIG. 5****,** only *Tth*PrimPol mediated RCA generated a major target fragment band at 9.3kb upon BsmBl digestion. In the case of RCA primed by a primer pair, minimal faithful amplification of the target sequence took place. This is demonstrated in the AGE by the presence of high molecular weight DNA material remaining in the well or in the smear sized 20kb with most not digested by BsmBl

Supplementation of the RCA reaction with random sequence heptamer stabiliser (NNNNNN(ddG)) at 600nM rescued faithful RCA amplification resulting in efficient RCA material digestibility with BsmBl and a prominent target sequence band at 9.3kb. Whilst the random sequence heptamer stabiliser used a terminal ddG, the use of stabilisers with a terminal ddA, ddC or ddT would be expected to yield similar results.

### Example 5: PolyG and PolyC stabilisers in the amplification of G:C homopolymer regions

### Method:

A DNA construct sized ~5300bp containing a CAG promoter with a 16bp long G:C homopolymer tract was amplified using rolling circle amplification (RCA) with QualiPhi^{®} (a chimeric form of Phi29 DNA polymerase, 4basebio). Priming of the template DNA strands was performed with *Tth*PrimPol enzyme that primes DNA in sequence-independent fashion. The reaction was incubated at 30°C for 20 hours under the same RCA conditions as provided for Example 1.

The DNA template utilized in the RCA consists of two complementary, denatured DNA strands where each strand can exhibit a different propensity for inhibiting amplification, e.g. the first strand may inhibit amplification while the second strand may not inhibit amplification. Therefore, in this example, each RCA reaction was also supplemented with 400nM of either Guanine Heptamer containing 3' dideoxy, chain terminating nucleotide (GGGGGG(ddG)) or Cytosine Heptamer containing 3' dideoxy, chain terminating nucleotide (CCCCCC(ddC)) to identify which strand may require the presence of complementary stabiliser molecules.

Amplified RCA material was digested with Bsal restriction enzyme to release the target monomeric sequence. The target product band was then gel excised and subjected to sequencing analysis.

### Results:

In case of the G:C homopolymer region, the differences in sequence length between faithfully and erroneously amplified target copies are not detectable by AGE (see **FIG. 6****).** However, sequencing data of the entire target region shows complete alignment between the theoretical target sequence and the amplified products across the entire sequence except for the CAG promoter. In the case of RCA reaction mediated by the *Tth*PrimPol enzyme in the absence of any stabilizer molecules, amplification across the G:C homopolymer tract is erroneous, resulting in G nucleotide deletions. Addition of the polyG (GGGGGG(ddG)) stabilizer to the primase-mediated RCA reaction rescues the faithful amplification resulting in target product with the exact sequence (indicating that there is an inhibitory region within the strand comprising the polyC homopolymer region). Addition of the polyC (CCCCCC(ddC)) stabilizer appears to have no impact on the amplification fidelity in this instance, which would be expected based on the inhibitory region being within the stand comprising a polyC homopolymer region.

### Example 6: RCA with a polyA stabiliser followed by adaptor ligation

### Method:

DNA construct sized ~3000bp, containing a 120bp poly-Adenine tail region was amplified using rolling circle amplification (RCA) with QualiPhi^{®} (a chimeric form of Phi29 DNA polymerase, 4basebio). Priming of the template DNA strands was performed with a pair of sequence-specific primers. The reaction was incubated at 30°C for 20 hours under the same RCA conditions as provided for Example 1. Additionally, the RCA was also supplemented with 200nM of Adenine Heptamer containing 3' dideoxy, chain terminating nucleotide AAAAAA(ddA). Amplified RCA material was digested by BsmBl restriction enzyme to assess target monomer size.

To generate adaptor-ligated product (opDNA), amplified DNAs were incubated with Type II restriction enzyme BsmBl, T4 DNA ligase and complementary adaptors to the 5' protruding ends generated by BsmBl on the amplified DNA. Digestion and ligation reaction was performed in reaction volume 1mL, containing 100 µl reaction buffer for DNA ligase 10x (500 mM Tris HCl pH 7.5, 100 mM MgCl₂, 100 mM DTT, 0.24 mg DNA, 0.88 µL BsmBl (29.7 µM), T4 DNA ligase (17.4 µM), 13.2 µg and 16.8 µg of first and second DNA adaptor molecule respectively, and incubation for 20 hours at 37°C. The first adaptor molecule comprised a closed loop (SEQ ID NO: 1) and the second adaptor molecule comprised a duplex comprising phosphorothioated nucleotides (SEQ ID NOs: 2 and 3), thus rendering the adaptor-ligated product exonuclease resistant.

Exonuclease clean-up reaction conditions: 2.23 µL of E. coli exonuclease I (17.6 µM) and 2.8 µL of E. coli exonuclease III (45.3 µM) are then added to remove remaining adaptors and open DNA molecules. Incubation time and temperature: 2 hours at 37°C.

The adaptor-ligated product was then further digested with BsaBI restriction enzyme to generate a smaller fragment containing mainly the A:T homopolymer sequence. This double digestion provides better resolution between bands of similar size in AGE e.g. a BsaBI fragment containing the full 120 A:T homopolymer sequence and a Sapl fragment containing a partial <120 A:T homopolymer sequence.

### Results:

**FIG. 7** shows amplified RCA material digested with BsmBI and the corresponding adaptor-ligated product (highlighted with arrows). The adaptor-ligated product (~3000bp), synthesized from the DNA amplified in presence of polyA stabilizer (AAAAAA(ddA)), adhered to all quality standards including appearance (clear, colourless and free of visible particulates), ratio of UV absorption (A260/A280 at 1.8 - 2.1), ratio of UV absorption (A260/A230 at 2.0 - 2.4), AGE gel identity (0.8%- clearly identifiable band of the expected size (bp)), sequence identity (a full sequence alignment), and protein contamination (≤20 µg/mg DNA (≤2%)).

The adaptor-ligated product was digested with BsaBI restriction enzyme to isolate the fragment containing the A:T homopolymer sequence. The BsaBI fragment with retained 120bp A:T homopolymer region is expected at ~250bp as illustrated in the AGE simulation. This is matched by the size of the BsaBl fragment generated when digesting the adaptor-ligated product (highlighted with a square border). This experiment demonstrates that the addition of the stabilizer molecule retains the homopolymer region in the final adaptor-ligated product and does not interfere with the adaptor ligation process.

The sequences discussed in the application are provided in the table below:

| **SEQ ID NO** | **Sequence (5'-3')** |
|---|---|
| **1** | AGGGCTAACATTTGTTGGCCACTCAGGCCAACAAATGTTAG |
| **2** | AAAAAAAAAAAAA*A*A*A*A*A |
| **3** | T*T*T*T*T*TTTTTTTTT |

## Claims

1. A method of amplifying DNA comprising:
a) providing a DNA template molecule comprising at least one inhibitory region; and
b) amplifying the DNA template molecule to generate an amplified DNA product, wherein the DNA template molecule is amplified in the presence of at least one type of single-stranded nucleic acid (ssNA) molecule that cannot be extended by a polymerase, wherein the at least one type of ssNA molecule anneals to the at least one inhibitory region, and wherein in the absence of the at least one type of ssNA molecule the inhibitory region inhibits complete and/or faithful amplification of the DNA template molecule.

2. The method of claim 1, wherein the DNA template molecule is amplified in the presence of a collection of ssNA molecules that cannot be extended by a polymerase, wherein the collection of ssNA molecules comprises multiple types of ssNA molecule.

3. The method of claim 2, wherein each type of ssNA molecule in the collection of ssNA molecules has a random sequence.

4. The method of any one of claims 1 to 3, wherein the collection of ssNA molecules comprises at least three types of ssNA molecule.

5. The method of any one of claims 1 to 4, wherein in the absence of the at least one type of ssNA molecule the at least one inhibitory region:
i) results in the amplified DNA product having a different sequence to the DNA template molecule, optionally wherein the amplified DNA product comprises an insertion and/or a deletion relative to the DNA template molecule; and/or
ii) reduces the yield of the amplified DNA product.

6. The method of any one of claims 1 to 5, wherein the amplification comprises isothermal amplification, preferably rolling circle amplification.

7. The method of any one of claims 1 to 6, wherein the amplified DNA product is concatemeric.

8. The method of any one of claims 1 to 7, wherein the method further comprises digesting the amplified DNA product with an endonuclease.

9. The method of any one of claims 1 to 8, wherein the ssNA molecules are between 6 and 8 nucleotides in length, preferably 7 nucleotides in length.

10. The method of any one of claims 1 to 9, wherein the at least one inhibitory region comprises:
i) a GC content of at least 60%;
ii) a repeated sequence;
ii) a homopolymeric sequence; and/or
iv) a sequence that is prone to forming an inhibitory structure, optionally wherein the inhibitory structure comprises (i) a structure that inhibits strand displacement by a polymerase; and/or (ii) a structure that inhibits a primer annealing to the DNA template molecule.

11. The method of any one of claims 1 to 10, wherein the at least one inhibitory region has a length of at least 8 bp.

12. The method of any one of claims 1 to 11, wherein the DNA template molecule is a single stranded DNA molecule.

13. The method of any one of claims 1 to 12, wherein the ssNA molecules:
a) cannot be extended by a polymerase due to a terminal 3' modification, optionally wherein the terminal 3' modification comprises a dideoxy modified nucleotide; and/or
b) the ssNA molecules are single-stranded DNA (ssDNA) molecules.

14. An adaptor-ligated DNA product comprising a linear double-stranded region comprising an inhibitory region, wherein the inhibitory region inhibits complete and/or faithful amplification of the linear double-stranded region, wherein the linear double-stranded region is ligated at a first end to a first adaptor molecule and ligated at a second end to a second adaptor molecule, wherein the first and second adaptor molecules confer exonuclease resistance to the adaptor-ligated DNA product and wherein the inhibitory region comprises: a polyG sequence and/or an insulator.

15. The adaptor-ligated DNA product of claim 14, wherein:
a) the linear double-stranded region is closed at a first end by the first adaptor molecule and closed at a second end by the second adaptor molecule;
b) the first and second adaptor molecules are nucleic acid molecules that comprise one or more nuclease-resistant nucleotides; or
c) the first adaptor molecule is a nucleic acid molecule that comprises one or more nuclease-resistant nucleotides, and wherein the linear double-stranded region is closed at the second end by the second adaptor molecule.
